# EUROPEAN PATENT APPLICATION

(11) **EP 3 156 497 A1**
(43) Date of publication of application: **19.04.2017**
(21) Application number: 15190102.2
(22) Date of filing: 16.10.2015
(51) Int. Cl.: C12Q 1/68

(54) **TRPV2 AS A BIOMARKER AND AS A THERAPEUTIC TARGET FOR MELANOMA**

(71) Applicant: Centre National de la Recherche Scientifique (C.N.R.S.), 75016 Paris (FR); INSERM (Institut National de la Santé et de la Recherche Médicale), 75013 Paris (FR); Université de Rennes 1, 35000 Rennes (FR)
(72) Inventor: PENNA, Aubin, 35700 Rennes (FR); SHOJI, Kenji, 35000 Rennes (FR)
(74) Representative: Cabinet Plasseraud

(57) **Abstract**

The present invention relates to TRPV2, a cation channel, whose expression at the mRNA level and at the protein levels, correlates with melanoma and with aggressive/invasive or metastatic melanoma. More specifically, the invention provides methods and kits for the diagnosis of melanoma and of aggressive/invasive or metastatic melanoma, and for the treatment of melanoma and the prevention of melanoma metastasis formation in melanoma patient.

## Description

### Background of the Invention

Melanocytes reside in the basal layer of the human epidermis providing UV-protecting melanin pigment for the surrounding keratinocytes. Malignant transformation of melanocytes causes the most aggressive form of skin cancer: melanoma. Despite the fact that cutaneous melanoma is rare, the worldwide incidence of the disease has increased over the past few decades, with more than 132,000 people diagnosed with the melanoma every year (World Health Organization). If melanoma is diagnosed early, it can be cured by surgical excision and this is what occurs in about 80% of the cases. However, once melanoma has begun to invade and metastasize, most patients reach a therapeutic dead end, since metastatic melanoma is refractory to current therapies. This results in high morbidity and mortality. Survival rate at five years goes down from 88% for localized stages to 18% for metastatic melanoma; and melanoma accounts for only about 4% of skin cancers, but for as many as 74% of all skin cancer deaths.

The prognosis of melanoma is based on histopathological criteria described in the American Joint Committee on Cancer (AJCC) melanoma staging system. These include the Breslow index, mitotic rate, ulceration status and extent of lymph node involvement (Balch et al., J. Clin. Oncol., 2009, 27: 6199-6206). Despite this staging system, the clinical behavior of melanoma is often unpredictable (Nagore et al., Melanoma Res., 2005, 15: 169-177) because melanoma is a group of diseases with various biological subtypes (Lomas et al., Front Biosci., 2008, 13: 5071-5093). Thus, understanding the mechanisms that drive tumor invasion and metastasis is critical for drug development aiming at preventing the spread of disease and related mortality.

In all cells, normal and pathological migration/invasion are calcium (Ca²⁺)-dependent responses since the involved apparatus comprise many Ca²⁺-sensitive effector molecules. However, even though the role of Ca²⁺ signaling has long been suspected in these processes, one component of the cellular migration/invasion machineries, namely ion channel proteins, was largely neglected until quite recently. This despite the fact that ion channels are a large family of proteins participating in virtually all cellular processes by initiating and shaping signaling pathways. Actually, due to their biological significance, accessibility to pharmacological modulation and exposure at the cell surface, ion channels represent major targets for current medicine. Over the last decade, it has become evident that Ca²⁺ channels act as novel and important regulators of specific steps in tumor progression covering all the hallmarks of cancer (Bödding, Cell Signal, 2007, 19: 617-624; Prevarskaya et al., Biochem. Biophys. Acta, 2007, 1772: 937-946; Gkika et al., Biochem. Biophys. Acta, 2009, 1793: 953-958; Santoni et al., Adv. Exp. Med. Biol., 2011, 704: 947-967; Santoni et al., Endocr. Metab. Immune Disord. Drug Targets, 2011, 11: 54-67; Fiorio Pla et al., Front Physiol., 2013, 4: 311; and Nielsen et al., Br. J. Pharmacol., 2014, 171: 5524-5540). Even so, they still remain a so far under-explored and under-exploited class of therapeutic targets for anti-cancer therapies. This can be partly explained by the paucity of data currently available on ion channel expression/roles in tumor progression of most cancers and by the lack of pharmacological tool to target specifically the subtypes of ion channels expressed in cancer cells

Thus, there still remains, in the art, an ongoing need for new strategies for the treatment and/or management of metastatic melanoma, and for the development of prognostic tools.

### Summary of the Invention

The present Inventors have observed that the cation channel, TRPV2 (VRL-1, GRC), is exceptionally highly over-expressed, at the mRNA level as well as at the protein level, in multiple melanoma cell lines, including metastatic melanoma cell lines. These results were corroborated in human melanoma tissues where an important difference in the TRPV2 protein expression levels was observed between melanoma samples and normal tissue samples, and between melanoma samples and metastatic melanoma samples. Furthermore, the TRPV2 expression levels were found to positively correlate with the invasive phenotype of the melanoma cell lines and of the tumor samples.

Accordingly, in a first aspect, the present invention relates to an *in vitro* method for diagnosing melanoma in a subject comprising steps of: (a) determining the expression level of TRPV2 in a biological sample obtained from the subject; and (b) comparing the expression level of TRPV2 measured in (a) with the expression level of TRPV2 measured in a biological sample obtained from at least one healthy subject, wherein an increased expression level of TRPV2 in the subject compared to the healthy subject is indicative of melanoma.

In certain embodiments, the *in vitro* method further comprises a step of: (c) comparing the expression level of TRPV2 measured in (a) with the expression level of TRPV2 measured in a biological sample obtained from at least one control subject diagnosed with non-metastatic melanoma, wherein an increased expression level of TRPV2 in the subject compared to the control subject is indicative of aggressive/invasive or metastatic melanoma.

The present invention also relates to an *in vitro* method for diagnosing aggressive/invasive or metastatic melanoma in a subject comprising steps of: (a) determining the expression level of TRPV2 in a biological sample obtained from the subject; and (b) comparing the expression level of TRPV2 measured in (a) with the expression level of TRPV2 measured in a biological sample obtained from at least one control subject diagnosed with non-metastatic melanoma, wherein an increased expression level of TRPV2 in the subject compared to the control subject is indicative of aggressive/invasive or metastatic melanoma.

The present invention also relates to an *in vitro* method for distinguishing between non-metastatic melanoma and metastatic melanoma in a subject or for determining the aggressiveness of melanoma in a subject, said method comprising steps of: (a) determining the expression level of TRPV2 in a biological sample obtained from the subject; (b) comparing the expression level of TRPV2 measured in (a) with the expression level of TRPV2 measured in a biological sample obtained from at least one healthy subject; and (c) comparing the expression level of TRPV2 measured in (a) with the expression level of TRPV2 measured in a biological sample obtained from at least one control subject diagnosed with non-metastatic melanoma, wherein an increased expression level of TRPV2 in the subject compared to the control subject is indicative of aggressive/invasive or metastatic melanoma, and wherein an expression level of TRPV2 in the subject comprised between the expression level of TRPV2 in the healthy subject and the expression level of TRPV2 in the control subject is indicative of non-metastatic melanoma.

In certain embodiments of the methods of diagnosis of the present invention, the biological sample is a skin biopsy sample. In certain embodiments of the methods of diagnosis of the present invention, the biological sample is a blood sample containing circulating melanoma cells.

In certain embodiments of the methods of diagnosis of the present invention, determining the expression level of TRPV2 in a biological sample may include measuring the amount of TRPV2 mRNA in the sample. Measuring the amount of TRPV2 mRNA in a biological sample may be performed using, for example, a nucleic acid hybridization technique, a nucleic acid reverse transcription technique, and/or a nucleic acid amplification technique.

In other embodiments of the methods of diagnosis of the present invention, determining the expression level of TRPV2 in a biological sample may include measuring the amount of TRPV2 protein in the sample. Measuring the amount of TRPV2 protein in a biological sample may be performed using, for example, an ELISA or immunoassay.

In the *in vitro* methods of the present invention, determining the expression level of TRPV2 in a biological sample comprises a step of: contacting the biological sample with a reagent that specifically reacts with TRPV2. A reagent that specifically reacts with TRPV2 may be selected among nucleic acid probes that specifically hybridize to TRPV2 mRNA transcripts, PCR primers that specifically amplify TRPV2 mRNA transcripts, antibodies that specifically recognize/bind to the TRPV2 protein or TRPV2-binding peptides that specifically bind to the TRPV2 protein. In certain embodiments, the reagent that specifically reacts with TRPV2 is labeled with a detectable moiety.

In a related aspect, the present invention relates to the use of TRPV2 as a biomarker of melanoma in a subject, and to the use of TRPV2 as a biomarker of aggressive/invasive or metastatic melanoma in a subject.

In another related aspect, the present invention concerns a reagent that specifically reacts with TRPV2 (as defined herein) for use in the *in vitro* diagnosis of melanoma, or the *in vitro* diagnosis of aggressive/invasive or metastatic melanoma.

In yet another related aspect, the present invention concerns a kit for use in the *in vitro* diagnosis of melanoma, or the *in vitro* diagnosis of aggressive/invasive or metastatic melanoma, wherein said kit comprises at least one reagent that specifically reacts with TRPV2 (as defined herein) and instructions to carry out a diagnosis method according to the present invention.

The present Inventors have also shown that TRPV2 over-expression in a non-invasive melanoma cell line confers an invasive phenotype to the cell line both *in vitro* and *in vivo.* In contrast, the invasive and migratory potential of metastatic melanoma cells was found to be reduced upon TRPV2 silencing. They also demonstrated that TRPV2 regulates melanoma cell migration through Calpain activation, focal adhesion remodelling and actin fibers polymerization. Thus, TRPV2 was found to be critical for melanoma tumor cell migration and invasion.

Accordingly, in a second aspect, the present invention relates to a TRPV2 therapeutic agent, or a pharmaceutical composition thereof, for use in the treatment of melanoma in a subject and/or for the prevention of melanoma metastasis formation in a subject. A pharmaceutical composition comprises a therapeutically effective amount of a TRPV2 therapeutic agent and at least one pharmaceutically acceptable carrier or excipient.

In a related aspect, the present invention concerns a method for treating melanoma in a subject in need thereof, comprising a step of: administering to said subject a therapeutically effective amount of a TRPV2 therapeutic agent, or a pharmaceutical composition thereof. In another related aspect, the present invention concerns a method for preventing melanoma metastasis formation in a subject in need thereof, comprising a step of: administering to said subject a therapeutically effective amount of a TRPV2 therapeutic agent, or a pharmaceutical composition thereof.

TRPV2 therapeutic agents that can be used in the treatment of melanoma include, but are not limited to, TRPV2-binding peptides and antibodies conjugated to a therapeutic moiety, and TRPV2 agonists and activators.

TRPV2 therapeutic agents that can be used in the prevention of melanoma metastasis formation in a patient include, but are not limited to, TRPV2 antagonists and RNA interfering agents.

In preferred embodiments, the subject is a human patient. Preferably, the human patient has been diagnosed with melanoma and/or has undergone treatment for melanoma.

These and other objects, advantages and features of the present invention will become apparent to those of ordinary skill in the art having read the following detailed description of the preferred embodiments.

### Definitions

Throughout the description, several terms are employed that are defined in the following paragraphs.

The term *"**biomarker**"* refers to a substance that is a distinctive indicator of a biological process, biological event and/or pathological condition. In the context of the present invention, the overexpression of TRPV2, at the mRNA level and/or protein level, is a distinctive indicator of melanoma, and a distinctive indicator of aggressive/invasive or metastatic melanoma.

The term ***"biological sample"*** is used herein in its broadest sense. A biological sample is generally obtained from a subject. A biological sample may be any biological tissue or fluid with which the biomarker of the present invention may be assayed. Examples of biological samples include, but are not limited to, blood samples which contain circulating melanoma cells *(e.g.,* whole blood, serum or plasma) and biological tissues such as skin tissue or fine needle biopsy samples of skin melanoma tumors, and archival samples with known diagnosis, treatment and/or outcome history. Biological samples may also include sections of tissues such as frozen sections taken for histological purposes. The term "biological sample" also encompasses any material derived by processing a biological sample. Derived materials include, but are not limited to, cells (or their progeny) isolated from the sample, as well as proteins or nucleic acid molecules (*e.g*., mRNA) extracted from the sample. Processing of a biological sample may involve one or more of: filtration, distillation, extraction, concentration, inactivation of interfering components, addition of reagents, and the like. In preferred embodiments of the invention, the biological sample is (or is derived from) melanoma tumor samples (primary, lymph nodes, metastases, short term culture) obtained from a subject.

In a diagnostic method according to the present invention, the biological sample to be tested is obtained from a subject (generally a subject suspected of having melanoma) and the results of the test are compared to those obtained for a biological sample obtained from a healthy subject. The terms "***normal***" and "***healthy***" are used herein interchangeably. When used to characterize a subject, they refer to a subject that is healthy, and in particular to a subject that does not suffer from melanoma, or any other cancer, or any other skin disease. Preferably, a healthy subject is not on medication that may affect Ca²⁺-channels. In certain embodiments, normal/healthy subjects have the same age, sex, and/or body mass index as compared to the subject from which the biological sample to be tested was obtained. The terms "normal" and "healthy" are also used herein to qualify a biological sample obtained from a healthy subject (or from a cohort of heathy subjects).

In certain embodiments, in a diagnostic method according to the present invention, the results obtained for the biological sample of the subject to be tested are compared with the results obtained for a biological sample of a control subject. In the context of the present invention, the term "***control***", when used herein to characterize a subject, refers to a subject that is healthy or to a patient who has been diagnosed with non-metastatic melanoma, or to a patient who has been diagnosed with a given stage/grade of melanoma, or yet to a patient who has been diagnosed with metastatic melanoma. The term ***"control sample"*** refers to one or more than on, sample that has been obtained from a control subject. It is known in the art that control samples are preferably obtained from a cohort of healthy subjects and/or of control patients. In some embodiments, the "control sample" is a pre-determined value, threshold or range.

The term "***antibody***", as used herein, refers to any immunoglobulin *(i.e.,* an intact immunoglobulin molecule, an active portion of an immunoglobulin molecule, etc.) that binds to a specific epitope. The term encompasses monoclonal antibodies and polyclonal antibodies. All derivatives (*e.g*., chimeric antibodies, humanized antibodies, single-chain antibodies) and fragments thereof *(e.g.,* Fab, Fv, scFv and Fd fragments), which maintain specific binding ability, are also included in the term. The term also covers any protein (or fusion protein) having a binding domain, which is homologous or largely homologous to an immunoglobulin-binding domain. These proteins may be derived from natural sources, or partly or wholly synthetically produced.

The terms *"**polypeptide**",* and *"**peptide**"* are used herein interchangeably, and refer to amino acid sequences of a variety of lengths, either in their neutral (uncharged) forms or as salts, and either unmodified or modified by glycosylation, side chain oxidation, or phosphorylation.

The term *"**specific binding**",* when used herein in reference to an antibody or a peptide, refers to an antibody or a peptide binding to a predetermined antigen. Typically, the antibody or peptide binds with an affinity of at least 1 x 10⁷ M⁻¹, and binds to the predetermined antigen with an affinity that is at least two-fold greater than the affinity for binding to a non-specific antigen (*e.g*., BSA, casein, etc).

The term *"**oligonucleotide**",* as used herein, refers to a short string of a deoxyribonucleotide or ribonucleotide polymer. Oligonucleotides can be used as amplification primers and/or detection probes. Such short stretches of nucleic acid sequences are often chemically synthesized. As will be appreciated by those skilled in the art, the length of an oligonucleotide can vary widely, often depending on its intended function or use. Generally, oligonucleotides comprise between about 5 and about 150 nucleotides, preferably between about 15 and about 100 nucleotides, more preferably between about 15 and about 50 nucleotides.

The term *"**hybridization**"* refers to the formation of complexes between nucleotide sequences which are sufficiently complementary to form complexes *via* Watson-Crick base pairing or non-canonical base pairing. When a primer "hybridizes" with a target sequence (template), such complexes (or hybrids) are sufficiently stable to serve the priming function required by, *e.g.,* the DNA polymerase, to initiate DNA synthesis. It will be appreciated that hybridizing sequences need not have perfect complementarity to provide stable hybrids. In many situations, stable hybrids will form where fewer than about 10% of the bases are mismatches. Those skilled in the art understand how to estimate and adjust the stringency of hybridization conditions such that sequences having at least a desired level of complementarity will stably hybridize, while those having lower complementarity will not. For examples of hybridization conditions and parameters see, *e.g.,* J. Sambrook et al., "Molecular Cloning: A Laboratory Manual", 2012, 4th Ed., Cold Spring Harbor Press: Plainview, NY; F.M. Ausubel, "Current Protocols in Molecular Biology", 1994, John Wiley & Sons: Secaucus, NJ. As used herein, the term "***specifically hybridizes***" means that cross-hybridization with mRNA other than TRPV2 mRNA is not significantly produced under ordinary hybridization conditions, preferably under stringent hybridization conditions (see, for example, the conditions disclosed in Sambrook et al., "Molecular Cloning: A Laboratory Manual", 2012, 4th Ed., Cold Spring Harbor Press: Plainview, NY).

As used herein, the term *"**amplification**"* refers to a method or process that increases the representation of a population of specific nucleic acid sequences in a sample. Amplification methods (such as polymerase chain reaction or reverse transcription polymerase chain reaction) are known in the art.

The terms "***target sequence**"* and ***"target nucleic acid**"* are used herein interchangeably. They refer to a nucleic acid sequence, the presence or absence of which is desired to be detected. In the context of the present invention, the target sequence preferably includes a nucleic acid sequence to which oligonucleotide primers will complex. The target sequence may also include a probe-hybridizing region with which a probe will form a stable hybrid under desired conditions.

The terms *"**primer**"* and *"**amplification primer"*** are used herein interchangeably. They refer to an isolated oligonucleotide which is capable of acting as a point of initiation of synthesis of a primer extension product, when placed under suitable conditions (*e.g*., buffer, salt, temperature and pH) in the presence of nucleotides and an agent for nucleic acid polymerization (*e.g.*, a DNA-dependent or RNA-dependent polymerase). The primer is preferably single-stranded for maximum efficiency in amplification, but may alternatively be double-stranded. If double-stranded, the primer may first be treated (*e.g*., denatured) to allow separation of its strands before being used to prepare extension products. Such a denaturation step is typically performed using heat, but may alternatively be carried out using alkali, followed by neutralization. A typical primer contains about 10 to about 50 nucleotides in length of a sequence substantially complementary to the target sequence. However, a primer can also contain additional sequences. For example, amplification primers used in Strand Displacement Amplification (SDA) preferably include a restriction endonuclease recognition at site 5' to the target binding sequence (see, for example, U.S. Pat. Nos 5,270,184 and 5,455,166). Nucleic Acid Sequence Based Amplification (NASBA), and Transcription-Mediated Amplification (TMA) primers preferably include an RNA polymerase promoter linked to the target binding sequence of the primer. Methods for linking such specialized sequences to a binding target sequence for use in a selected amplification reaction are well-known in the art.

The term "***amplification conditions***", as used herein, refers to conditions that promote annealing and/or extension of primer sequences. Such conditions are well-known in the art and depend on the amplification method selected. Thus, for example, in a PCR reaction, amplification conditions generally comprise thermal cycling, *i.e.,* cycling of the reaction mixture between two or more temperatures. In isothermal amplification reactions, amplification occurs without thermal cycling although an initial temperature increase may be required to initiate the reaction. Amplification conditions encompass all reaction conditions including, but not limited to, temperature and temperature cycling, buffer, salt, ionic strength, pH and the like.

As used herein, the term "***amplification reaction reagents***" refers to reagents used in nucleic acid amplification reactions and may include, but are not limited to, buffers, reagents, enzymes having reverse transcriptase and/or polymerase activity or exonuclease activity; enzyme cofactors such as magnesium or manganese; salts; and deoxynucleotide triphosphates (dNTPs) such as deoxyadenosine triphosphate (dATP), deoxyguanosine triphosphate (dGTP), deoxycytidine triphosphate (dCTP), deoxythymidine triphosphate (dTTP) and deoxyuridine triphosphate (dUTP). Amplification reaction reagents may readily be selected by one skilled in the art depending on the amplification method used.

The terms *"**probe**"* and *"**detection probe"*** are used herein interchangeably and refer to an oligonucleotide capable of selectively hybridizing to at least a portion of a target sequence under appropriate conditions (*e.g*., a portion of a target sequence that has been amplified). In general, a probe sequence is identified as being either "complementary" *(i.e.,* complementary to the coding or sense strand (+)), or "reverse complementary" *(i.e.,* complementary to the anti-sense strand (-)). In certain embodiments, a detection probe is labeled with a detectable agent or moiety.

The terms *"**labeled**",* "***labeled with a detectable agent***" and "***labeled with a detectable moiety***" are used herein interchangeably. These terms are used to specify that an entity *(e.g.,* an antibody or a peptide) can be visualized, for example, following binding to another entity *(e.g.,* a protein biomarker). Preferably, a detectable agent or moiety is selected such that it generates a signal which can be measured and whose intensity is related to the amount of bound entity. In array-based methods, a detectable agent or moiety is also preferably selected such that it generates a localized signal, thereby allowing spatial resolution of the signal from each spot on the array. Methods for labeling proteins and polypeptides are well known in the art. Labeled polypeptides (*e.g*., antibodies) can be prepared by incorporation of or conjugation to a label, that is directly or indirectly detectable by spectroscopic, photochemical, biochemical, immunochemical, electrical, optical, or chemical means, or any suitable means. Suitable detectable agents include, but are not limited to, various ligands, radionuclides, fluorescent dyes, chemiluminescent agents, microparticles, enzymes, colorimetric labels, magnetic labels, and haptens.

The term ***"directly detectable***", when used herein in reference to a label or detectable moiety, means that the label or detectable moiety does not require further reaction or manipulation to be detectable. For example, a fluorescent moiety is directly detectable by fluorescence spectroscopy methods. The term *"**indirectly detectable***", when used herein in reference to a label or detectable moiety, means that the label or detectable moiety becomes detectable after further reaction or manipulation. For example, a hapten becomes detectable after reaction with an appropriate antibody attached to a reporter, such as a fluorescent dye.

The term "***isolated***" when referring herein to an oligonucleotide, an antibody or a peptide means an oligonucleotide, an antibody or a peptide which, by virtue of its origin or manipulation, is separated from at least some of the components with which it is naturally associated or with which it is associated when initially obtained or prepared. By "isolated", it is alternatively or additionally meant that the oligonucleotide, antibody or peptide is produced or synthesized by the hand of man.

As used herein, the term ***"subject"*** refers to a human or another mammal (*e.g*., primate, dog, cat, goat, horse, pig, mouse, rat, rabbit, and the like) that can develop a melanoma, but may or may not have the pathology. Non-human subjects may be transgenic or otherwise modified animals. In many embodiments of the present invention, the subject is a human being. In such embodiments, the subject is often referred to as an *"**individual**"* or a "***patient***". The terms "subject", "individual" and "patient" do not denote a particular age, and thus encompass newborns, children, teenagers, and adults. The term "patient" more specifically refers to an individual suffering from a disease, *e.g.,* a melanoma.

The term ***"melanoma patient",*** as used herein, refers to a patient who has been diagnosed with melanoma. The diagnostic may have been performed using any method known in the art and/or using a method according to the present invention. The term also includes individuals who are undergoing or have previous undergone therapy for melanoma.

The terms ***"aggressive"*** and ***"invasive"*** are used herein interchangeably. When used herein to characterize a cancer, in particular a melanoma, they refer to the proclivity of a tumor for expanding beyond its boundaries into adjacent tissue. Invasive cancer can be contrasted with organ-confined cancer wherein the tumor is confined to a particular organ or to a particular location in an organ. The invasive property of a tumor is often accompanied by the elaboration of proteolytic enzymes, such as collagenases, that degrade matrix material and basement membrane material to enable the tumor to expand beyond the confines of the capsule, and beyond confines of the particular tissue in which that tumor is located.

The term ***"metastasis",*** as used herein, refers to the spread of tumor cells from one organ or tissue to another location. The term also refers to tumor tissue that forms in a new location as a result of metastasis. A "metastatic cancer" is a cancer that spreads from its original, or primary, location, and may also be referred to as a "secondary cancer" or "secondary tumor". Generally, metastatic tumors are named for the tissue of the primary tumor from which they originate.

As used herein, the term *"**inhibit**"* means to prevent something from happening, to delay occurrence of something happening, and/or to reduce the extent or likelihood of something happening. Thus, the terms "inhibiting metastasis", "inhibiting metastases" and "inhibiting the formation of metastases", which are used herein interchangeably, are intended to encompass preventing, delaying, and/or reducing the likelihood of occurrence of metastases as well as reducing the number, growth rate, size, etc... of metastases.

The term *"**treatment**"* is used herein to characterize a method or process that is aimed at (1) delaying or preventing the onset of a disease or condition (here melanoma or metastatic melanoma); (2) slowing down or stopping the progression, aggravation, or deterioration of the symptoms of the disease or condition; (3) bringing about amelioration of the symptoms of the disease or condition; or (4) curing the disease or condition. A treatment may be administered after initiation of the disease or condition, for a therapeutic action. Alternatively, a treatment may be administered prior to the onset of the disease or condition, for a prophylactic or preventive action. In this case, the term "***prevention***" is used.

A *"**pharmaceutical composition"*** is defined herein as comprising an effective amount of a TRPV2 therapeutic agent and at least one pharmaceutically acceptable carrier or excipient.

As used herein, the term ***"therapeutically effective amount"*** refers to any amount of a compound, agent, or composition that is sufficient to fulfil its intended purpose(s), *e.g.,* a desired biological or medicinal response in a cell, tissue, system or subject. For example, in certain embodiments of the present invention, the purpose(s) may be: to prevent, delay, and/or reduce the likelihood of occurrence of metastases and/or reduce the number, growth rate, size, etc... of metastases. The purpose(s) may also include lengthening the time to progression of the disease in a melanoma patient.

The term *"**pharmaceutically acceptable carrier or excipient"*** refers to a carrier medium which does not interfere with the effectiveness of the biological activity of the active ingredient(s) and which is not excessively toxic to the host at the concentration at which it is administered. The term includes solvents, dispersion media, coatings, antibacterial and antifungal agents, isotonic agents, and adsorption delaying agents, and the like. The use of such media and agents for pharmaceutically active substances is well known in the art (see for example "Remington's Pharmaceutical Sciences", E.W. Martin, 18th Ed., 1990, Mack Publishing Co.: Easton, PA, which is incorporated herein by reference in its entirety).

The terms ***"approximately"*** and ***"about",*** as used herein in reference to a number, generally include numbers that fall within a range of 10% in either direction of the number (greater than or less than the number) unless otherwise stated or otherwise evident from the context (except where such number would exceed 100% of a possible value).

### Detailed Description of Certain Preferred Embodiments

As mentioned above, the present invention identifies TRPV2, as a biomarker of melanoma, and as a biomarker of aggressive/invasive or metastatic melanoma. The present invention also identifies TRPV2 as a therapeutic target for the treatment of melanoma and for the prevention of melanoma metastasis formation. Thus, the invention contains at least two aspects: a diagnostic aspect and a therapeutic aspect.

### I - Diagnostic Aspect

### A. TRPV2 as a Biomarker of Melanoma and Melanoma Aggressiveness

The present invention provides the identity of a gene, the *TRPV2* gene, whose expression at the transcriptome and proteome levels was found to be indicative of melanoma and of melanoma aggressiveness.

TRPV2 belongs to the family of calcium ion channels called Transient Receptor Potentials (TRPs). Transient receptor potential (TRP) ion channels are emerging as a new set of membrane proteins involved in a vast array of cellular processes and regulated by a large number of physical and chemical stimuli. The vanilloid TRP subfamily (TRPV) consists of six members, and at least four of them (TRPV 1-TRPV4) have been reported as being related to thermal sensation. TRPV2 is one of the least characterized members of the TRP subfamily. Although initially characterized as a noxious heat sensor, TRPV2 now seems to have little to do with temperature sensing, but has a much more complex physiological profile (Peràlvarez-Marin et al., FEBS J., 2013, 280(21): 5471-5487).

As used herein, the term *"TRPV2"* refers to the gene that encodes the protein called Transient Receptor Potential cation channel subfamily V member 2 or "TRPV2". In embodiments of the invention that concern a particular mammal species, the term *"TRPV2"* refers to the gene of that particular mammal species, and the term "TRPV2" prefers to a protein encoded by said gene. The *TRPV2* gene of a variety of mammal species and correponding TRPV2 protein have been sequenced and are known in the art. TRPV2 presents a high conservation level of identity within mammalina orthologs; and consists of a large N-cytoplasmic domain (∼390 residues) followed by six transmembrane segments (∼250 residues) containing a pore-forming loop, and a C-terminal cytoplasmic domain (∼100 residues).

In embodiments of the present invention concerning a human subject, the term *"TRPV2"* refers to the human *TRPV2* gene that is located on the small (p) arm of chromosome 17 at position 11.2 (Gene ID: 51393) and that encodes the human protein, TRPV2. The mRNA sequence of *TRPV2* is given by GenBank Accession Number RefSeq(mRNA): NM_016113.4, and the corresponding protein sequence is given by GenBank Accession Number RefSeq(protein): NP_057197.2. One skilled in the art knows that TRPV2 splice variants exist as well as clinical variants such as clinical variants of malignant melanoma *(e.g.,* NM_016113.4(TRPV2):c.815G>A (p.Gly272Glu)).

### B. Detection and Quantification of the Biomarker TRPV2

The methods of the present invention comprise determining the expression level of TRPV2 in a biological sample obtained from the subject to be tested. The level of *TRPV2* expression can be assessed at the protein level or at the mRNA level.

### Biological Samples

The methods described herein may be applied to the testing of any biological sample allowing *TRPV2* to be assayed at the mRNA level or the protein level. The biological sample is generally obtained from a subject to be tested. The subject is generally a mammal (e.g., primate, dog, cat, horse, mouse, rat, and the like). In certain embodiments of the present invention, the subject is a dog (melanoma being frequent in some dog species). In certain preferred embodiments of the present invention, the subject to be tested is a human being.

In certain embodiments, the TRPV2 biomarker is detected and its expression level is quantified in a sample of skin tissue. In other embodiments, the TRPV2 biomarker is detected and its expression level is quantified in a sample of blood (e.g., whole blood, serum or plasma) containing circulating melanoma tumor cells (Haber and Velculescu, Cancer Discovery, 2014, 4(6): 650-651; Lianidou et al., Crit. Rev. Clin. Lab. Sci., 2014, 51(3): 160-171; Mateo et al., Genome Biol., 2014, 15(8): 448).

In certain embodiments, the methods of diagnosis of the present invention are performed on the biological sample without any prior major manipulation of the sample. In other embodiments, the inventive methods are performed on nucleic acid extracts or protein extracts prepared from the biological sample.

For example, RNA may be extracted from skin samples or blood samples and analyzed using a method of the invention. Methods of RNA extraction are well known in the art (see, for example, J. Sambrook et al., "Molecular Cloning: A Laboratory Manual", 1989, 2nd Ed., Cold Spring Harbour Laboratory Press: New York). Most methods of RNA isolation from bodily fluids or tissues are based on the disruption of the tissue in the presence of protein denaturants to quickly and effectively inactivate RNases. Generally, RNA isolation reagents comprise, among other components, guanidium thiocyanate and/or beta-mercaptoethanol, which are known to act as RNase inhibitors. Isolated total RNA may then be further purified from the protein contaminants and concentrated by selective ethanol precipitations, phenol/chloroform extractions followed by isopropanol precipitation (see, for example, P. Chomczynski and N. Sacchi, Anal. Biochem., 1987, 162: 156-159) or cesium chloride, lithium chloride or cesium trifluoroacetate gradient centrifugations.

Numerous versatile kits can be used to extract RNA *(i.e.,* total RNA or mRNA) from human bodily fluids or tissues, that are commercially available from, for example, Ambion, Inc. (Austin, TX), Amersham Biosciences (Piscataway, NJ), BD Biosciences Clontech (Palo Alto, CA), BioRad Laboratories (Hercules, CA), GIBCO BRL (Gaithersburg, MD), and Giagen, Inc. (Valencia, CA). User Guides that describe in great detail the protocol to be followed are usually included in all these kits. Sensitivity, processing time and cost may be different from one kit to another. One of ordinary skill in the art can easily select the kit(s) most appropriate for a particular situation.

In certain embodiments, after extraction, mRNA is amplified, and transcribed into cDNA, which can then serve as template for multiple rounds of transcription by the appropriate RNA polymerase. Amplification methods are well known in the art (see, for example, A.R. Kimmel and S.L. Berger, Methods Enzymol. 1987, 152: 307-316; J. Sambrook et al., "Molecular Cloning: A Laboratory Martial", 1989, 2nd Ed., Cold Spring Harbour Laboratory Press: New York; *"*Short Protocols in Molecular Biology", F.M. Ausubel (Ed.), 2002, 5th Ed., John Wiley & Sons). Reverse transcription reactions may be carried out using non-specific primers, such as an anchored oligo-dT primer, or random sequence primers, or using a target-specific primer complementary to the RNA being monitored, or using thermostable DNA polymerases (such as avian myeloblastosis virus reverse transcriptase or Moloney murine leukemia virus reverse transcriptase).

The inventive methods may alternatively be performed on a protein extract from the biological sample. Preferably, the protein extract contains the total protein content. Methods of protein extraction are well known in the art (see, for example "Protein Methods", D.M. Bollag et al., 2nd Ed., 1996, Wiley-Liss; *"*Protein Purification Methods: A Practical Approach", E.L. Harris and S. Angal (Eds.), 1989; *"*Protein Purification Techniques: A Practical Approach", S. Roe, 2nd Ed., 2001, Oxford University Press; *"*Principles and Reactions of Protein Extraction, Purification, and Characterization", H. Ahmed, 2005, CRC Press: Boca Raton, FL). Different kits can be used to extract proteins from bodily fluids and tissues that are commercially available from, for example, BioRad Laboratories (Hercules, CA), BD Biosciences Clontech (Mountain View, CA), Chemicon International, Inc. (Temecula, CA), Calbiochem (San Diego, CA), Pierce Biotechnology (Rockford, IL), and Invitrogen Corp. (Carlsbad, CA). One of ordinary skill in the art can easily select the kit(s) most appropriate for a particular situation. After the protein extract has been obtained, the protein concentration of the extract is preferably standardized to a value being the same as that of the control sample in order to allow signals of the protein markers to be quantified. Such standardization can be performed using photometric or spectrometric methods or gel electrophoresis.

Although the present Description generally relates to *in vitro* diagnosis, it will be recognized by one skilled in the art that the diagnosis methods of the present invention may alternatively be performed *in vivo.*

### Contacting Biological Samples with a Reagent that Specifically Reacts with TRPV2

In the methods of the present invention, the level of *TRPV2* expression can be assessed at the protein level or at the nucleic acid level. Methods for determining the level of expression of a gene at either the nucleic acid or protein level are well known in the art and include, but are not limited to, immunoblots (western blots), northern blots, Southern blots, enzyme linked immunosorbent assay (ELISA), immunoprecipitation, immunofluorescence, flow cytometry, immunohistochemistry, nucleic acid hybridization techniques, nucleic acid reverse transcription methods, and nucleic acid amplification methods.

Generally, a method according to the invention comprises a step of contacting the sample obtained from the subject to be tested with a reagent that specifically reacts with TRPV2.

As used herein, the term "a reagent that specifically reacts with TRPV2" refers to a reagent used to detect and determine the expression level of the biomarker of the present invention, TRPV2. Examples of suitable reagents include, but are not limited to, nucleic acid probes that specifically hybridize to TRPV2 mRNA transcripts, PCR primers that specifically amplify TRPV2 mRNA transcripts, antibodies that specifically recognize and bind to the TRPV2 protein or TRPV2-binding peptides that specifically bind to the TRPV2 protein. In certain embodiments, the reagent that specifically reacts with TRPV2 is labeled with a detectable moiety.

Using the TRPV2 sequence information provided herein (mRNA sequence or protein sequence) or any other TRPV2 sequence information available in the art, it is within the capabilities of one skilled in the art to develop reagents that specifically react with TRPV2 at the mRNA level or at the protein level. Similarly, one skilled in the art knows how to develop reagents that specifically react with the TRPV2 channels of other mammal species, at the mRNA level or at the protein level. Since *TRPV2* is conserved in many species, the reagents that specifically react with TRPV2 may be designed to be usable for more than one mammal species.

As used herein, the term "contacting the biological sample with a reagent that specifically reacts with TRPV2" typically includes, but is not limited to, mixing or incubating the biological sample with a reagent that specifically reacts with TRPV2. In preferred embodiments, the step of contacting the biological sample is performed for a time and under conditions allowing the reagent to react with TRPV2. As will be recognized by one skilled in the art, when the method is carried out *in vivo,* "contacting the biological sample" may include administering (e.g., injecting) to the subject to be tested the reagent that specifically reacts with TRPV2.

It is within the abilities of one skilled in the art to determine the optimal conditions allowing the reagent to react with TRPV2 mRNA or TRPV2 protein present in a biological sample and the TRPV2 mRNA or TRPV2 protein to be detected and quantified. In certain embodiments, the conditions are selected such that a complex forms between the antibody reagent and the TRPV2 protein present in the biological sample. In other embodiments, the conditions are selected such that a complex forms between the TRPV2-binding peptide and the TRPV2 protein present in the biological sample. In yet other embodiments, the conditions are selected such that nucleic acid probes can hybridize to the TRPV2 mRNA transcripts present in the biological sample, in particular under stringent hybridization conditions. In still other embodiments, the conditions are selected such that PCR primers can amplify TRPV2 mRNA transcripts.

### Determination of TRPV2 Protein Expression Levels

In certain embodiments, *TRPV2* expression is determined at the protein level. Determination of protein expression levels in the practice of the inventive methods may be performed by any suitable method known in the art (see, for example, E. Harlow and A. Lane, "Antibodies: A Laboratories Manual", 1988, Cold Spring Harbor Laboratory: Cold Spring Harbor, NY).

*Agents that specifically react with TRPV2 protein*. In general, the expression level of a protein in a biological sample obtained from a subject or patient is determined by contacting the biological sample with a reagent that specifically reacts with the TRPV2 protein biomarker; determining, in the biological sample, the level of TRPV2 protein that binds to the reagent; and comparing the protein level determined in the biological sample with the protein level measured in a control sample, and eventually with the level of one or more reference proteins. In these embodiments, the term "reagent that specifically reacts with TRPV2 protein" refers to an entity such as a peptide or antibody that specifically recognizes and binds to the TRPV2 biomarker. An entity "specifically recognizes and/or binds" to a protein if it reacts/interacts at a detectable level with the protein but does not react/interact with polypeptides containing unrelated sequences or sequences of different polypeptides.

Thus, in certain embodiments, the reagent that specifically reacts with TRPV2 protein is an antibody specific for TRPV2. Suitable antibodies for use in methods of the invention include monoclonal and polyclonal antibodies, immunologically active fragments (e.g., Fab or (Fab)₂ fragments), antibody heavy chains, humanized antibodies, antibody light chains, and chimeric antibodies. Antibodies, including monoclonal and polyclonal antibodies, fragments and chimeras, may be prepared using methods known in the art (see, for example, R.G. Mage and E. Lamoyi, in "Monoclonal Antibody Production Techniques and Applications", 1987, Marcel Dekker, Inc.: New York, pp. 79-97; G. Kohler and C. Milstein, Nature, 1975, 256: 495-497; D. Kozbor et al., J. Immunol. Methods, 1985, 81: 31-42; and R.J. Cote et al., Proc. Natl. Acad. Sci. 1983, 80: 2026-203; R.A. Lerner, Nature, 1982, 299: 593-596; A.C. Nairn et al., Nature, 1982, 299: 734-736; A.J. Czernik et al., Methods Enzymol. 1991, 201: 264-283; A.J. Czernik et al., Neuromethods: Regulatory Protein Modification: Techniques & Protocols, 1997, 30: 219-250; A.J. Czernik et al., Neuroprotocols, 1995, 6: 56-61; H. Zhang et al., J. Biol. Chem. 2002, 277: 39379-39387; S.L. Morrison et al., Proc. Natl. Acad. Sci., 1984, 81: 6851-6855; M.S. Neuberger et al., Nature, 1984, 312: 604-608; S. Takeda et al., Nature, 1985, 314: 452-454). Antibodies to be used in the methods of the invention can be purified by methods well known in the art (see, for example, S.A. Minden, "Monoclonal Antibody Purification", 1996, IBC Biomedical Library Series: Southbridge, MA). For example, antibodies can be affinity-purified by passage over a column to which TRPV2, or a fragment thereof, is bound. The bound antibodies can then be eluted from the column using a buffer with a high salt concentration.

Instead of being prepared, antibodies to be used in the methods of the present invention may be obtained from scientific or commercial sources. Examples of commercially available anti-TRPV2 antibodies include, but are not limited to, anti-TRPV2 antibodies commercialized by Abcam, Santa-Cruz Biotechnology, AbD Serotec, ThermoFisher Scientific, Antibodies-online, LSBio, ABGENT, GeneTex, Sigma-Aldrich, Pierce Antibodies Products, OriGene, BioCompare, and Alomone Labs.

In certain embodiments, the reagent that specifically reacts with TRPV2 protein is a TRPV2-binding peptide. Protein-binding peptides and methods for designing or identifying protein-binding peptides are known in the art (Zhang et al., Nature Biotechnology, 2000, 18: 71-74; Sood et al., J. Mol. Biol., 2006, 357(3): 917-921; London et al., Structure, 2010, 18(2): 188-199; Sammond et al., J. Am. Chem. Soc., 2011, 133(12): 4190-4192). TRPV2-binding peptides generally contain between 9 and 35 amino acid residues, for example, between 9 and 13, 10 and 15, 15 and 20, 18 and 25, 24 and 30, 27 and 35 or 32 and 36 amino acids. They may be coupled to other sequences and/or moieties.

*Labeled Binding Agents.* Preferably, the binding agent *(e.g.,* an anti-TRPV2-antibody or a TRPV2-binding peptide) is directly or indirectly labeled with a detectable moiety. The role of a detectable agent is to facilitate the detection step by allowing visualization of the complex formed by reaction or association between the binding agent and the protein biomarker. Preferably, the detectable agent is selected such that is generates a signal which can be measured and whose intensity is related (preferably proportional) to the amount of protein biomarker present in the sample being analyzed. Methods for labeling biological molecules such as polypeptides and antibodies are well-known in the art (see, for example, *"*Affinity Techniques. Enzyme Purification: Part B", Methods in Enzymol., 1974, Vol. 34, W.B. Jakoby and M. Wilneck (Eds.), Academic Press: New York, NY; and M. Wilchek and E.A. Bayer, Anal. Biochem., 1988, 171: 1-32).

Any of a wide variety of detectable agents can be used in the practice of the present invention. Suitable detectable agents include, but are not limited to: various ligands, radionuclides, fluorescent dyes, chemiluminescent agents, microparticles (such as, for example, quantum dots, nanocrystals, phosphors, and the like), enzymes (such as, for example, those used in an ELISA, *i.e.,* horseradish peroxidase, beta-galactosidase, luciferase, alkaline phosphatase), colorimetric labels, magnetic labels, and biotin, dioxigenin or other haptens, and proteins for which antisera or monoclonal antibodies are available.

In certain embodiments, the binding agent *(e.g.,* an anti-TRPV2 antibody or a TRPV2-binding peptide) may be immobilized on a carrier or support *(e.g.,* a bead, a magnetic particle, a latex particle, a microtiter plate well, a cuvette, or other reaction vessel). Examples of suitable carrier or support materials include agarose, cellulose, nitrocellulose, dextran, Sephadex, Sepharose, liposomes, carboxymethyl cellulose, polyacrylamydes, polystyrene, gabbros, filter paper, magnetite, ion-exchange resin, plastic film, plastic tube, glass, polyamine-methyl vinyl-ether-maleic acid copolymer, amino acid copolymer ethylene-maleic acid copolymer, nylon, silk, and the like. A binding agent may be indirectly immobilized using a secondary binding agent specific for the first binding agent *(e.g.,* a mouse antibody specific for a protein biomarker may be immobilized using an sheep anti-mouse IgG Fc fragment specific antibody coated on the carrier or support). The secondary binding agent may be coupled to a detectable tag, such as for example, an enzyme, fluorophore, or chromophore.

Anti-TRPV2 antibodies can be used to determine TRPV2 protein expression level in various immunoassays. Examples of such assays are radioimmunoassay, enzyme immunoassays (*e.g*., ELISA), immunofluorescence, immunoprecipitation, latex agglutination, hemagglutination, and histochemical tests, which are conventional methods well-known in the art. As will be appreciated by one skilled in the art, the immunoassay may be competitive or non-competitive. Methods of detection and quantification of the signal generated by the complex formed by reaction or association of the binding agent with the protein biomarker will depend on the nature of the assay and of the detectable moiety (*e.g*., fluorescent moiety).

In embodiments of the present invention where the diagnosis is performed *in vivo,* antibody and peptide labels or markers for *in vivo* imaging of protein include those detectable by X-radiography, NMR (Nuclear Magnetic Resonance) or ESR (Election Spin Resonance). For X-radiography, suitable labels include radioisotopes such as barium or cesium, which emit detectable radiation but are not overtly harmful to the subject. Suitable markers for NMR and ESR include those with a detectable characteristic spin, such as deuterium, which may be incorporated into the antibody by labeling of nutrients for the relevant hybridoma. A protein-specific antibody or antibody fragment which has been labeled with an appropriate detectable imaging moiety, such as a radioisotope (for example, ¹³¹I, ¹¹²In, ^{99m}Tc), a radio-opaque substance, or a material detectable by nuclear magnetic resonance, is introduced (for example, parenterally, subcutaneously, or intraperitoneally) into the subject to be tested. It will be understood in the art that the size of the subject and the imaging system used will determine the quantity of imaging moiety needed to produce diagnostic images. The labeled antibody or antibody fragment will then preferentially accumulate at the location of cells which overexpress TRPV2 (Burchiel et al., "Immunopharmacokinetics of Radiolabeled Antibodies and Their Fragments", Chapter 13 in Tumor Imaging: The Radiochemical Detection of Cancer, Burchiel and Rhodes, Eds., Masson Publishing Inc. (1982)).

### Determination of TRPV2 Nucleic Acid Expression Levels

In certain embodiments, *TRPV2* expression is determined at the nucleic acid level, in particular at the mRNA level. Nucleic acid-based techniques for assessing expression are well known in the art and include, for example, determining the level of *TRPV2* mRNA in a biological sample (*e.g*., skin sample). Isolated mRNA can be used in hybridization or amplification assays that include, but are not limited to, Southern or northern analyses, polymerase chain reaction analyses (PCR or reverse transcription polymerase chain reaction) and probe arrays.

In general, the expression level of TRPV2 mRNA in a biological sample obtained from a subject or patient is determined by contacting the biological sample with a reagent that specifically reacts with TRPV2 mRNA; determining, in the biological sample, the level of TRPV2 mRNA; and comparing the protein level determined in the biological sample with the TRPV2 mRNA level measured in a control sample. In these embodiments, the term "reagent that specifically reacts with TRPV2 mRNA" refers to an entity that specifically binds to TRPV2 mRNA. One method for the detection of mRNA levels involves contacting the isolated total mRNA with a nucleic acid probe that specifically hybridizes to TRPV2 mRNA. Another method for the detection of mRNA levels involves contacting the isolated total mRNA with nucleic acid primers that specifically hybridize to the TRPV2 mRNA and amplify at least a portion of the TRPV2 mRNA to produce amplicons that can be detected and quantified.

The primers and probes useful in the practice of the present invention may be oligonucleotides comprising at least 15 nucleotides.

When the oligonucleotide is used as a primer, its length is usually 15 to 100 nucleotides, preferably 17 to 30 nucleotides. However, the primer is not particularly limited as long as it is capable of amplifying at least a portion of a TRPV2 mRNA transcript. In certain embodiments, a reagent according to the present invention is a pair of primers comprising: a 5' (upstream) primer (or forward primer) that hybridizes with the 5' end of the nucleic acid sequence to be amplified and a 3' (downstream) primer (or reverse primer) that hybridizes with the complement of the sequence to be amplified. Such primer pairs are particularly useful in PCR amplification reactions.

In addition to a nucleotide sequence identical or complementary to the nucleotide sequence of the target sequence, the primer may comprise any nucleotide sequence added thereto. Furthermore, the primer may be modified. For example, primers may be labeled with a detectable agent or moiety *(e.g.,* a fluorescent substance or a binding affinity substance such as biotin or digoxin).

These primer pairs can be used according to any nucleic acid amplification technique that employs two or more oligonucleotides to amplify a target sequence. Amplification products produced using a primer pair may be detected using any of a variety of detection methods well known in the art. For example, amplification products may be detected using agarose gel electrophoresis and visualization by ethidium bromide staining and exposure to ultraviolet (UV) light or by sequence analysis of the amplification product, or any other method known in the art.

When the oligonucleotide is used as a probe, its length is usually 5 to 200 nucleotides, preferably 7 to 100 nucleotides, more preferably 7 to 50 nucleotides. The probe is not particularly limited as long as it is capable of specifically hybridizing to a portion of a TRPV2 mRNA transcript. From the sequence information provided above, one skilled in the art knows how to the design and prepare suitable probes.

Generally probes have a complimentary nucleic acid sequence that selectively hybridizes to the target nucleic acid sequence. In order for a probe to hybridize to a target sequence, the hybridization probe must have sufficient identity with the target sequence, *i.e.,* at least 70% (e.g., 80%, 90%, 95%, 98% or more) identity to the target sequence. The probe sequence must also be sufficiently long so that the probe exhibits selectivity for the target sequence over non-target sequences.

An oligonucleotide useful in the practice of the present invention (primer or probe) may include one or more sequences which can serve as spacers, linkers, sequences for labeling or binding to an enzyme, which may impart added stability or susceptibility to degradation process or other desirable property to the oligonucleotide.

*Preparation of Primers and Probes.* The primers and probes that can be used in the practice of the present invention may be prepared using any of a variety of methods well known in the art (see, for example, J. Sambrook et al., "Molecular Cloning: A Laboratory Manual", 2012, 4th Ed., Cold Spring Harbour Laboratory Press: New York, NY; *"*PCR Protocols: A Guide to Methods and Applications", 1990, M.A. Innis (Ed.), Academic Press: New York, NY; P. Tijssen "Hybridization with Nucleic Acid Probes-Laboratory Techniques in Biochemistry and Molecular Biology (Parts I and II)", 1993, Elsevier Science; *"*PCR Strategies", 1995, M.A. Innis (Ed.), Academic Press: New York, NY; and *"*Short Protocols in Molecular Biology", 2002, F.M. Ausubel (Ed.), 5th Ed., John Wiley & Sons: Secaucus, NJ). For example, oligonucleotides may be prepared by chemical synthesis and polymerization based on a template as described, *e.g.,* in S.A. Narang et al., Meth. Enzymol. 1979, 68: 90-98; E.L. Brown et al., Meth. Enzymol. 1979, 68: 109-151; E.S. Belousov et al., Nucleic Acids Res. 1997, 25: 3440-3444; D. Guschin et al., Anal. Biochem. 1997, 250: 203-211; M.J. Blommers et al., Biochemistry, 1994, 33: 7886-7896; and K. Frenkel et al., Free Radic. Biol. Med. 1995, 19: 373-380; and U.S. Pat. No. 4,458,066).

For example, oligonucleotides may be prepared using an automated, solid-phase procedure based on the phosphoramidite approach. Such syntheses may be performed on oligo synthesizers such as those commercially available from Perkin Elmer/Applied Biosystems, Inc. (Foster City, CA), DuPont (Wilmington, DE) or Milligen (Bedford, MA). Alternatively, oligonucleotides can be custom made and ordered from a variety of commercial sources well-known in the art, including, for example, the Midland Certified Reagent Company (Midland, TX), ExpressGen, Inc. (Chicago, IL), Operon Technologies, Inc. (Huntsville, AL), BioSearch Technologies, Inc. (Novato, CA), and many others.

Purification of oligonucleotides of the invention, where necessary or desired, may be carried out by any of a variety of methods well known in the art. Purification of oligonucleotides is typically performed either by native acrylamide gel electrophoresis, by anion-exchange HPLC as described, for example, by Pearson et al. (J. Chrom., 1983, 255: 137-149) or by reverse phase HPLC (McFarland et al., Nucleic Acids Res., 1979, 7: 1067-1080).

The sequence of oligonucleotides can be verified using any suitable sequencing method including, but not limited to, chemical degradation (Maxam et al., Methods of Enzymology, 1980, 65: 499-560), matrix-assisted laser desorption ionization time-of-flight (MALDI-TOF) mass spectrometry (Pieles et al., Nucleic Acids Res., 1993, 21: 3191-3196), mass spectrometry following a combination of alkaline phosphatase and exonuclease digestions (Wu et al., Anal. Biochem., 2001, 290: 347-352), and the like.

*Labeling of Primers and*/*or Probes.* In certain embodiments, detection probes or amplification primers or both probes and primers are labeled with a detectable agent or moiety before being used in amplification/detection assays. In some preferred embodiments, the detection probes are labeled with a detectable agent. The role of a detectable agent is to allow visualization and detection of amplified target sequences (amplicons). Preferably, the detectable agent is selected such that it generates a signal which can be measured and whose intensity is related (e.g., proportional) to the amount of amplification products in the sample being analyzed.

The association between an oligonucleotide (*e.g*., detection probe) and a detectable agent can be covalent or non-covalent. Labeled detection probes can be prepared by incorporation of, or conjugation to, a detectable moiety. Labels can be attached directly to the nucleic acid sequence or indirectly (*e.g*., through a linker). Linkers and spacer arms of various lengths are known in the art and are commercially available, and can be selected to reduce steric hindrance, or to confer other useful or desired properties to the resulting labeled molecules (see, for example, Mansfield et al., Mol. Cell Probes, 1995, 9: 145-156).

Methods for labeling nucleic acid molecules are well-known in the art. For a review of labeling protocols and label detection techniques, see, for example, Kricka, Ann. Clin. Biochem. 2002, 39: 114-129; van Gijlswijk et al., Expert Rev. Mol. Diagn. 2001, 1: 81-91; and Joos et al., J. Biotechnol. 1994, 35: 135-153. Standard nucleic acid labeling methods include: incorporation of radioactive agents, direct attachments of fluorescent dyes or of enzymes; chemical modifications of nucleic acid molecules making them detectable immunochemically or by other affinity reactions; and enzyme-mediated labeling methods, such as random priming, nick translation, PCR and tailing with terminal transferase (for a review on enzymatic labeling, see, for example, Temsamani et al., Mol. Biotechnol. 1996, 5: 223-232). Other nucleic acid labeling systems include, but are not limited to: ULS (Universal Linkage System), which is based on the reaction of monoreactive cisplatin derivatives with the N7 position of guanine moieties in DNA (Heetebrij et al., Cytogenet. Cell. Genet. 1999, 87: 47-52), psoralen-biotin, which intercalates into nucleic acids and upon UV irradiation becomes covalently bonded to the nucleotide bases (Levenson et al., Methods Enzymol. 1990, 184: 577-583; and Pfannschmidt et al., Nucleic Acids Res. 1996, 24: 1702-1709), photoreactive azido derivatives (Neves et al., Bioconjugate Chem. 2000, 11: 51-55), and DNA alkylating agents (Sebestyen et al., Nat. Biotechnol. 1998, 16: 568-576).

Any of a wide variety of detectable agents can be used in the practice of the present invention. Suitable detectable agents include, but are not limited to, various ligands, radionuclides (such as, for example, ³²P, ³⁵S, ³H, ¹⁴C, ¹²⁵I, ¹³¹I, and the like); fluorescent dyes (see below); chemiluminescent agents (such as, for example, acridinium esters, stabilized dioxetanes, and the like); spectrally resolvable inorganic fluorescent semiconductor nanocrystals *(i.e.,* quantum dots), metal nanoparticles (e.g., gold, silver, copper and platinum) or nanoclusters; enzymes (such as, for example, those used in an ELISA, *i.e.,* horseradish peroxidase, beta-galactosidase, luciferase, alkaline phosphatase); colorimetric labels (such as, for example, dyes, colloidal gold, and the like); magnetic labels (such as, for example, Dynabeads™); and biotin, dioxigenin or other haptens and proteins for antisera or monoclonal antibodies are available.

In certain embodiments, the invention detection probes are fluorescently labeled. Numerous known fluorescent labeling moieties of a wide variety of chemical structures and physical characteristics are suitable for use in the practice of this invention. Suitable fluorescent dyes include, but are not limited to, fluorescein and fluorescein dyes *(e.g.,* fluorescein isothiocyanine or FITC, naphthofluorescein, 4',5'-dichloro-2',7'-dimethoxy-fluorescein, 6-carboxyfluorescein or FAM), carbocyanine, merocyanine, styryl dyes, oxonol dyes, phycoerythrin, erythrosin, eosin, rhodamine dyes (*e.g*., carboxytetramethylrhodamine or TAMRA, carboxyrhodamine 6G, carboxy-X-rhodamine (ROX), lissamine rhodamine B, rhodamine 6G, rhodamine Green, rhodamine Red, tetramethylrhodamine or TMR), coumarin and coumarin dyes (e.g., methoxycoumarin, dialkylaminocoumarin, hydroxycoumarin and amino-methylcoumarin or AMCA), Oregon Green Dyes *(e.g.,* Oregon Green 488, Oregon Green 500, Oregon Green 514), Texas Red, Texas Red-X, Spectrum Red™, Spectrum Green™, cyanine dyes *(e.g.,* Cy-3™, Cy-5™, Cy-3.5™, Cy-5.5™), Alexa Fluor dyes *(e.g.,* Alexa Fluor 350, Alexa Fluor 488, Alexa Fluor 532, Alexa Fluor 546, Alexa Fluor 568, Alexa Fluor 594, Alexa Fluor 633, Alexa Fluor 660 and Alexa Fluor 680), BODIPY dyes (e.g., BODIPY FL, BODIPY R6G, BODIPY TMR, BODIPY TR, BODIPY 530/550, BODIPY 558/568, BODIPY 564/570, BODIPY 576/589, BODIPY 581/591, BODIPY 630/650, BODIPY 650/665), IRDyes *(e.g.,* IRD40, IRD 700, IRD 800), and the like. For more examples of suitable fluorescent dyes and methods for linking or incorporating fluorescent dyes to nucleic acid molecules see, for example, *"*The Handbook of Fluorescent Probes and Research Products", 9th Ed., Molecular Probes, Inc., Eugene, OR. Fluorescent dyes as well as labeling kits are commercially available from, for example, Amersham Biosciences, Inc. (Piscataway, NJ), Molecular Probes Inc. (Eugene, OR), and New England Biolabs Inc. (Berverly, MA).

Rather than being directly detectable themselves, some fluorescent groups (donors) transfer energy to another fluorescent group (acceptor) in a process of fluorescent resonance energy transfer (FRET), and the second group produces the detectable fluorescent signal. In these embodiments, the oligonucleotide detection probe may, for example, become detectable when hybridized to an amplified target sequence. Examples of FRET acceptor/donor pairs suitable for use in the present invention include, but are not limited to, fluorescein/tetramethylrhodamine, IAEDANS/FITC, IAEDANS/5-(iodoacetomido)fluorescein, B-phycoerythrin/Cy-5, FAM/TAMRA and EDANS/Dabcyl.

The use of physically linked fluorescent reporter/quencher molecule pairs is also within the scope of the invention. The use of such systems in TaqMan™ assays or as Molecular Beacons is well-known in the art. With the TaqMan™ assay format, products of the amplification reaction can be detected as they are formed in a so-called "real-time" manner. As a result, amplification product/probe hybrids are formed and detected while the reaction mixture is under amplification conditions.

In some embodiments of the present invention, the PCR detection probes are TaqMan™-like probes that are labeled at the 5'-end with a fluorescent moiety and at the 3'-end with a quencher moiety. Suitable fluorophores and quenchers for use with TaqMan™-like probes are known in the art. Examples of quenchers include, but are not limited to DABCYL (*i.e*., 4-(4'-dimethylaminophenylazo)-benzoic acid) succinimidyl ester, diarylrhodamine carboxylic acid, succinimidyl ester (or QSY-7), and 4',5'-dinitrofluorescein carboxylic acid, succinimidyl ester (or QSY-33) (all available, for example, from Molecular Probes), quencher1 (Q1; available from Epoch Biosciences, Bothell, WA), or "Black hole quenchers" BHQ-1, BHQ-2, and BHQ-3 (available from BioSearch Technologies, Inc., Novato, CA).

A "tail" of normal or modified nucleotides can also be added to oligonucleotide probes for detectability purposes. A second hybridization with a nucleic acid molecule complementary to the tail and containing one or more detectable labels allows visualization of the amplicon/probe hybrids (see, for example, the system commercially available from Enzo Biochem, Inc. New York, NY). Another example of an assay with which the inventive oligonucleotides are useful is a signal amplification method such as that described in U.S. Pat. No. 5,124,246. In that method, the signal is amplified through the use of amplification multimers, polynucleotides which are constructed so as to contain a first segment that hybridizes specifically to the "tail" added to the oligonucleotide probes, and a multiplicity of identical second segments that hybridize specifically to a labeled probe. The degree of amplification is theoretically proportional to the number of iterations of the second segment. The multimers may be either linear or branched. Branched multimers may be in the shape of a fork or a comb.

The selection of a particular nucleic acid labeling technique will depend on the situation and will be governed by several factors, such as the ease and cost of the labeling method, the quality of sample labeling desired, the effects of the detectable moiety on the hybridization reaction *(e.g.,* on the rate and/or efficiency of the hybridization process), the nature of the amplification method used, the nature of the detection system, the nature and intensity of the signal generated by the detectable label, and the like.

*Immobilization of Probes.* In certain embodiments, a detection probe is immobilized on a solid support. For example, the solid support may be a bead, a particle, a microplate well, an array, a cuvette, a tube, a membrane, a gel, a resin, and the like.

In certain embodiments, an inventive probe is immobilized by being either covalently or non-covalently bound to the surface of a solid carrier or support. The term "solid support" refers to a material to which nucleic acid molecules can be immobilized. The solid support is not particularly limited as long as it is capable of immobilizing nucleic acid molecules. Thus, in the practice of the present invention, the solid support may be any solid support known in the art to which the probe can be operably affixed. "Operably affixed" refers to the probe being affixed in a manner permitting the formation of a complex between the affixed probe and the amplicons (or the target sequence) present in the biological sample tested. Examples of suitable carrier or support materials include, but are not limited to, agarose, cellulose, nitrocellulose, dextran, Sephadex, Sepharose, carboxymethyl cellulose, polyacrylamides, polystyrene, polyvinyl chloride, polypropylene, gabbros, magnetic ion-exchange resin, glass, polyamine-methyl-vinyl-ether-maleic acid copolymer, amino acid copolymer, ethylene-maleic acid copolymer, nylon, silk, and the like. In certain embodiments, immobilization of a probe to a solid carrier or support includes gel electrophoresis followed by transfer to a membrane (typically nitrocellulose or PVDF) in a process called western blotting (or immunoblot) well known in the art.

Methods for generating arrays are known in the art and include, *e.g.,* photolithographic methods, mechanical methods, pin-based methods, and bead-based techniques. Oligonucleotide probes forming an array may be attached to a substrate by any number of techniques, including, without limitation, (i) *in situ* synthesis *(e.g.,* high-density oligonucleotide arrays) using photolithographic techniques; (ii) spotting/printing at medium to low density on glass, nylon or nitrocellulose; (iii) by masking, and (iv) by dot-blotting on a nylon or nitrocellulose hybridization membrane. Oligonucleotides can be immobilized *via* a linker, including by covalent, ionic, or physical linkage. Linkers for immobilizing nucleic acids and polypeptides, including reversible or cleavable linkers, are known in the art.

### Expression Levels of TRPV2

Once the TRPV2 expression level has been determined in the biological sample obtained from a subject to be tested, it may be compared to the TRPV2 expression level determined in one or more control or reference samples.

As known in the art, comparison of expression levels is preferably performed after the expression levels obtained have been corrected for both differences in the amount of sample assayed and variability in the quality of the sample used (*e.g*., volume of sample, amount of protein extracted or number of cells stained, or amount and quality of mRNA tested). Correction may be carried out using any suitable method well-known in the art. For example, the protein concentration of a sample may be standardized using photometric or spectrometric methods or gel electrophoresis or *via* cell counting before the sample is analyzed. For analyses performed on nucleic acid molecules, correction may be carried out by normalizing the levels against reference genes (*e.g*., housekeeping genes such as, for example, the *B2M* (β-2-microglobulin) gene and the *HPRT1* (hypoxanthine phosphonbosyltransferase) gene) in the same sample.

Normalized expression levels of the TRPV2 biomarker determined in a biological sample to be tested according to a method of the invention may then be compared to the normalized expression levels of the TRPV2 biomarker determined in one or more control biological samples. In certain embodiments, the control biological sample originates from a healthy subject. In other embodiments, the control biological sample originates from a patient diagnosed with non-metastatic melanoma. In yet other embodiments, the control biological sample originates from a patient diagnosed with a given grade of malignant melanoma (*e.g*., Grade I, Grade II, Grade III or Grade IV). The subject to be tested using a method of the invention and the control subject must be of the same mammalian species. In certain preferred embodiments, the subject to be tested and the control subject are of the same sex, and of similar age, and preferably have similar body mass index. In embodiments concerning human subjects, the subject to be tested and the control subject also preferably have similar skin pigmentations.

In certain preferred embodiments, normalized expression levels of the TRPV2 biomarker determined in a biological sample to be tested according to a method of the invention are compared to the average of normalized expression levels of the TRPV2 biomarker determined for biological samples obtained from a significant number of control subjects.

Comparison of the TRPV2 expression level measured in the biological sample provided by the subject to be tested with the TRPV2 expression level measured in one or more biological samples from control subjects allows to establish a diagnostic. One skilled in the art knows how to select the controls and comparisons to be performed in order to reach a diagnosis.

### C. Melanoma Diagnosis using the Biomarker TRPV2

In certain embodiments, the (mRNA or protein) expression level of TRPV2 in a biological sample obtained from the subject to be tested is compared with the (mRNA or protein) expression level of TRPV2 measured in a biological sample obtained from at least one healthy subject. An increased expression level of TRPV2 in the subject compared to the healthy subject is indicative of melanoma. In other words, if the amount of TRPV2 mRNA or protein (preferably after normalization) measured in the biological sample obtained from the subject to be tested is found to be higher than the amount of TRPV2 mRNA or protein (preferably after normalization) measured in the biological sample obtained from the healthy subject, then melanoma is diagnosed. The (mRNA or protein) expression level of TRPV2 in the biological sample obtained from the subject to be tested may then be compared with the (mRNA or protein) expression level of TRPV2 in a biological sample obtained from at least one control subject diagnosed with non-metastatic melanoma. An increased expression level of TRPV2 in the subject compared to the control subject indicates that the melanoma is aggressive/invasive or that the melanoma is metastatic. In other words, if the amount of TRPV2 mRNA or protein (preferably after normalization) measured in the biological sample obtained from the subject to be tested is found to be higher than the amount of TRPV2 mRNA or protein (preferably after normalization) measured in the biological sample obtained from the control subject, then the melanoma of the subject is diagnosed as aggressive/invasive or metastatic melanoma.

In other embodiments, the (mRNA or protein) expression level of TRPV2 measured in a biological sample obtained from the subject to be tested is compared with the (mRNA or protein) expression level of TRPV2 measured in a biological sample obtained from at least one control subject diagnosed with non-metastatic melanoma. An increased expression level of TRPV2 in the subject compared to the control subject indicates that the melanoma is aggressive/invasive or that the melanoma is metastatic.

In yet other embodiments, the (mRNA or protein) expression level of TRPV2 that the melanoma is in a biological sample obtained from the subject to be tested is compared with the expression level of TRPV2 measured in a biological sample obtained from at least one healthy subject; and with the expression level of TRPV2 measured in a biological sample obtained from at least one control subject diagnosed with non-metastatic melanoma. Aggressive/invasive or metastatic melanoma is diagnosed if the amount of TRPV2 mRNA or protein (preferably after normalization) measured in the biological sample obtained from the subject to be tested is found to be higher than the amount of TRPV2 mRNA or protein (preferably after normalization) measured in the biological sample obtained from the control subject. Non-metastatic melanoma is diagnosed if the amount of TRPV2 mRNA or protein (preferably after normalization) measured in the biological sample obtained from the subject to be tested is found to be comprised between the amount of TRPV2 mRNA or protein (preferably after normalization) measured in the sample obtained from the healthy subject and the amount of TRPV2 mRNA or protein (preferably after normalization) measured in the sample obtained from the control subject.

As used herein, by the terms "increase" or "higher" refer to an increase (or augmentation) of at least 5%, at least about 10%, at least about 20%, at least 25%, at least 30%, at least 40%, at least about 50%, at least about 75%, at least about 80%, at least about 100%, at least about 200% (*i.e.,* 2-fold), or at least about 500% (*i.e.,* 5-fold), or at least about 10,000% *(i.e.,* 10-fold) or more over the level of expression under control conditions.

As used herein, by the terms "decrease" or "lower" refer to a decrease (or reduction) of at least 5%, at least about 10%, at least about 25%, at least about 50%, at least about 75%, at least about 80%, at least about 90% or less of the level of expression under control conditions.

Using diagnostic methods described herein, skilled physicians may, based on the diagnosis reached, select and prescribe treatments adapted to each patient (for example patients who are highly likely to develop metastases versus patients who have a better prognosis). Selection of an appropriate therapeutic regimen for a given patient may be made based solely on the diagnosis provided by the inventive methods. Alternatively, the physician may also consider other clinical or pathological parameters used in existing methods to diagnose melanoma and to grade the disease.

### D. Diagnostic Kits

In another aspect, the present invention provides kits comprising materials useful for carrying out a diagnostic method of the invention. The diagnostic procedures described herein may be performed by analytical laboratories, research laboratories and practitioners. The invention provides kits that can be used in these different settings.

Materials and reagents for performing a diagnostic method of the present invention may be assembled together in a kit. In certain embodiments, an inventive kit comprises at least one reagent that specifically reacts with the TRPV2 biomarker. Thus, in certain embodiments, a kit comprises at least one reagent that specifically reacts with TRPV2 mRNA and that allows the expression level of the *TRPV2* gene to be detected at the mRNA level *(e.g.,* a probe or a pair of primers, as described above). In other embodiments, a kit comprises at least one reagent that specifically reacts with TRPV2 protein and that allows the expression level of the *TRPV2* gene to be detected at the protein level *(e.g.,* an antibody or TRPV2-binding peptide, as described above).

In addition, an inventive kit may further comprise at least one reagent for the detection of a protein biomarker-antibody complex formed between an antibody included in the kit *(i.e.,* an anti-TRPV2 antibody) and the protein biomarker *(i.e.,* TRPV2) present in a biological sample obtained from a subject. Such a reagent may be, for example, a labeled antibody that specifically recognizes antibodies from the species tested *(e.g.,* an anti-human IgG). If the antibodies are provided attached to the surface of an array, a kit of the invention may comprise only one reagent for the detection of biomarker-antibody complexes (*e.g*., a fluorescently-labeled anti-human antibody).

Depending on the procedure, the kit may further comprise one or more of: extraction buffer and/or reagents, amplification buffer and/or reagents, hybridization buffer and/or reagents, immunodetection buffer and/or reagents, labeling buffer and/or reagents, and detection means. Protocols for using these buffers and reagents to perform different steps of the diagnostic procedure may be included in the kit. Other reagents can be included in separate containers and provided with the kit, *e.g*., positive control samples or compounds, negative control samples or compounds, buffers, cell culture media, specific detection probes, and the like.

The reagents may be supplied in a solid (*e.g.*, lyophilized) or liquid form. The kits of the present invention may optionally comprise different containers (*e.g.*, vial, ampoule, test tube, flask or bottle) for each individual buffer and/or reagent. Each component will generally be suitable as aliquoted in its respective container or provided in a concentrated form. Other containers suitable for conducting certain steps of the disclosed methods may also be provided. The individual containers of the kit are preferably maintained in close confinement for commercial sale.

A kit according to the present invention may further comprise instructions for using the kit according to a method of the invention. Instructions for using the kit according to a method of the invention may comprise instructions for processing the biological sample obtained from the subject to be tested, and/or instructions for contacting the sample with the reagent that specifically reacts with TRPV2, and/or instructions for labelling primers, and/or probes, and/or instructions for performing the test, and/or instructions for interpreting the results. Optionally associated with the container(s) can be a notice or package insert in the form prescribed by a governmental agency regulating the manufacture, use or sale of pharmaceutical or biological products, which notice reflects approval by the agency of manufacture, use or sale for human administration.

An identifier, *e.g.,* a bar code, radio frequency, ID tags, etc., may be present in or on the kit. The identifier can be used, for example, to uniquely identify the kit for purposes of quality control, inventory control, tracking movement between workstations, etc.

### II - Therapeutic Aspect

Since TRPV2 was found to be expressed at very high levels almost exclusively in melanoma and to be critical for melanoma tumor cell migration and invasion (see Examples below), TRPV2 can also be used as a therapeutic target. Thus, the present invention concerns a TRPV2 therapeutic agent, or a pharmaceutical composition thereof, for use in the treatment of melanoma in a subject and/or for use in the prevention of melanoma metastases formation in a subject. The present invention also relates to a method for treating melanoma in a subject in need thereof, comprising a step of: administering to said subject a therapeutically effective amount of a TRPV2 therapeutic agent, or a pharmaceutical composition thereof, and to a method for preventing melanoma metastasis formation in a subject in need thereof, comprising a step of: administering to said subject a therapeutically effective amount of a TRPV2 therapeutic agent, or a pharmaceutical composition thereof.

### A. TRPV2 Therapeutic Agents

As used herein, the term "TRPV2 therapeutic agent" refers to a molecule or a compound that interacts or interferes with TRPV2 normal activity to produce a therapeutic effect. The therapeutic effect may result from any of a large variety of action mechanisms. For example, a TRPV2 therapeutic agent may block the activity of TRPV2, lead to TRPV2 over-activation, inhibit the expression of *TRPV2,* destroy, degrade or cleave TRPV2 mRNAs or protein, block TRPV2 mRNA translation, or target a therapeutic moiety to TRPV2-overexpressing cells. Thus, examples of TRPV2 therapeutic agents include, but are not limited to, TRPV2 agonists, TRPV2 blockers or inhibitors (such as TRPV2 antagonists or RNA interfering agents), and TRPV2-binding compounds conjugated to therapeutic moieties.

### TRPV2 Agonists or Activators

TRPV2 agonists or direct or indirect activators that are suitable for use in the therapeutic applications of the present invention may be any TRPV2 compound or molecule that exhibits an agonistic activity on TRPV2 channels or that trigger TRPV2 channels activity. Examples of TRPV2 agonists include probenecid, delta-9-tetrahydrocannabinol (THC), cannabinol, cannabidiol, 2-aminoethoxydiphenyl borate and its derivatives, such as diphenylboronic anhydride. In the context of the present invention, TRPV2 agonists may be used to specifically eliminate melanoma tumor cells and/or to facilitate chemotherapeutics administration. Examples of indirect activators include, but are not limited to, lysophosphatidylcholine, and lysophosphatidylinositol.

### TRPV2 Blockers or Inhibitors

TRPV2 blockers or inhibitors suitable for use in the therapeutic applications of the present invention include TRPV2 antagonists and RNA interfering agents.

Examples of TRPV2 antagonists include synthetic molecules such as tranilast (N-(3,4-dimethoxycinnamoyl)-anthranilic acid), an antiallergic drug which has been described as an ion channel blocker, and derivatives thereof. Other TRPV2 blockers include SKF96365, amiloride, tetraethylammoniul, Ruthenium red, fampridin, TRIM, citral and lanthanium (La³⁺).

SKF96365, amiloride, tetraethylammonium, Ruthenium red, fampridin, TRIM, citral and lanthanum (La³⁺).

Other TRPV2 inhibitors can be identified, for example, by first screening for either synthetic or naturally occurring molecules that bind to the TRPV2 protein, and then in a second step, by testing those selected molecules in cellular assays for inhibition of TRPV2 activity. Such screening for molecules that bind to TRPV2 can easily be performed on a large scale, *e.g.,* by screening candidate molecules from libraries of synthetic and/or natural molecules. Thus, TRPV2 inhibitors may be found among synthetic organic chemicals, natural fermentation products, substances extracted from microorganisms, plants or animals, or peptides.

Other TRPV2 inhibitors include blocking anti-TRPV2 antibodies, in particular blocking anti-TRPV2 monoclonal antibodies.

Other agents that can be used as TRPV2 inhibitors include RNA interfering agents. As used herein, the term "RNA interference" (or "RNAi") has its art understood meaning and refers to a biological process in which RNA molecules silence, inhibit or down-regulate gene expression by causing the destruction, degradation and/or cleavage of specific mRNA molecules or by blocking the translation thereof. As known in the art, RNA interference is now exploited in therapy. Indeed, RNAi can be initiated by the hand of man, for example, to silence the expression of target genes. The terms "RNAi agent" and "RNA interfering agent" are used herein interchangeably. They refer to any RNA molecule that is capable of specifically inhibiting or down-regulating the expression of a target gene (here the *TRPV2* gene). By "silencing, inhibiting or down-regulating expression of a target gene", it is meant that the expression of the target gene, or level of RNA molecules or equivalent RNA molecules encoding one or more proteins or protein subunits, or activity of one or more proteins or protein subunits, is reduced below that observed in the absence of the RNAi agent.

An RNAi agent may be any single-stranded RNA (*e.g*., mature miRNA, ssRNAi oligonucleotides, ssDNAi oligonucleotides) or double-stranded RNA *(i.e.,* duplex RNA such as siRNA, Dicer-substrate dsRNA, shRNA, aiRNA, or pre-miRNA) that is capable of reducing or inhibiting the expression of a target gene or sequence (*e.g*., by mediating the degradation or inhibiting the translation of mRNAs which are complementary to the interfering RNA sequence) when the RNAi agent is in the same cell as the target gene or sequence. The term "RNAi agent" thus refers to the single-stranded RNA that is complementary to a target mRNA sequence (or circRNA sequence) or to the double-stranded RNA formed by two complementary strands or by a single, self-complementary strand. An RNAi agent may have substantial or complete identity to the target gene mRNA (or circRNA) or sequence, or may comprise a region of mismatch *(i.e.,* a mismatch motif). Consequently, the term "RNAi agent" refers to a RNA molecule comprising a strand having a sequence sufficiently complementary to a target mRNA (or circRNA) sequence to direct target-specific RNA interference (RNAi) thereby inhibiting or down-regulating the expression of the target gene.

An RNAi agent can comprise naturally occurring RNA, synthetic RNA, or recombinantly produced RNA, as well as altered RNA that differs from naturally-occurring RNA by the addition, deletion, substitution and/or alteration of one or more nucleotides. Such alterations can include addition of non-nucleotide material, such as to the end of the molecule or to one or more internal nucleotides of the RNAi, including modifications that make the RNAi agent resistant to nuclease digestion. An RNAi agent may be administered in free (naked) form or by the use of delivery systems that enhance stability and/or targeting, *e.g*., liposomes, or incorporated into other vehicles, such as hydrogels, cyclodextrins, biodegradable nanocapsules, bioadhesive microspheres, or proteinaceous vectors (WO 00/53722), or in combination with a cationic peptide (US 2007/275923). RNAi agents may also be administered in the form of their precursors or encoding DNAs.

In certain preferred embodiments of the present invention, an RNAi agent is a siRNA (small interfering RNA), a shRNA (short hairpin RNA), a micro-RNA (micro RNA), or an aiRNA (asymmetric interfering RNA). The development of any type of RNAi agent capable of specifically silencing, inhibiting or down-regulating the expression of a given target gene (here the *TRPV2* gene) is within the capabilities of one skilled in the art. The use of RNAi agents, such as siRNAs, shRNAs, micro-RNAs or aiRNAs, to inhibit gene expression is well known in the art.

The terms "small interfering RNA" and *"siRNA"* are used herein interchangeably. They refer to an RNA (or RNA analog) comprising between about 10-50 nucleotides (or nucleotide analogs) which is capable of directing or mediating RNA interference. Preferably, a siRNA comprises between about 15-30 nucleotides or nucleotide analogs, more preferably between about 16-25 nucleotides (or nucleotide analogs), even more preferably between about 18-23 nucleotides (or nucleotide analogs), and even more preferably between about 19-22 nucleotides (or nucleotide analogs) (e.g., 19, 20, 21 or 22 nucleotides or nucleotide analogs).

The terms "asymmetric interfering RNA" and "aiRNA" refer to an siRNA which is characterized by the length asymmetric between the two RNA strands.

The term "short hairpin RNA" (or shRNA) refers to a sequence of RNA having one or more loop structures and a stem comprising self-complementary sense and antisense regions, wherein the antisense region comprises a sequence complementary to a region of the target mRNA. A short hairpin RNA is cleaved by the cellular machinery into siRNA. The stem can be 29, 28, 27, 26, 25, 24, 23, 22, 21, 20, 19, 18, 17, 16, 15, 14, 13, 12, 11 or 10 base pairs in length, for example between 19 and 25 base pairs, or between 19 and 21 base pairs in length. The loop can vary in length. For example, the loop may be 5, 6, 7, 8, 9, 10, 11, 12 or 13 nucleotides in length. The hairpin structure can also contain 3' or 5' overhang portions. For example, the overhang is a 3' or a 5' overhang 0, 1, 2, 3, 4 or 5 nucleotides in length.

The term "micro-RNA" (or "miRNA"), as used herein, has its art understood meaning. MiRNAs are a major group of noncoding RNAs that are known to regulate almost a third of all the coding genes. They are small (∼ 20-25 nucleotides long) endogenously formed repressors of gene expression. miRNAs usually bind to the 3' untranslated region (3'UTR) of the target RNA transcripts (mRNAs or circRNAs) and are capable of inducing posttranscriptional gene regulation by blocking translation or by degrading the target RNAs, or by doing both. miRNAs can also be chemically synthesized. In contrast to siRNA, which has perfect complementarity to the target RNA transcripts, miRNA binds imperfectly to the target RNA transcripts.

TRPV2 agonists (TRPV2 antagonists and RNA interfering agents) may be useful in the prevention of melanoma metastasis formation.

### TRPV2-Binding Compounds Conjugated to Therapeutic Moieties

As will be recognized by one skilled in the art, the TRPV2-binding compounds (anti-TRPV2 antibodies or TRPV2-binding peptides, as described above) that specifically recognize and/or bind to TRPV2 overexpressed at the surface of melanoma cells, may also be used for targeting therapeutic moieties to these cells. Therefore, a further aspect of the present invention relates to a TRPV2-binding compound conjugated to at least one therapeutic moiety for use in the treatment of melanoma.

A TRPV2-binding compound is "conjugated" to a therapeutic moiety when it is functionally linked or attached (*e.g*., by chemical coupling, fusion, non-covalent association or otherwise) to the therapeutic moiety. Methods for the preparation of such conjugated antibodies or peptides are known in the art. (see, for example, *"*Affinity Techniques. Enzyme Purification: Part B", Methods in Enzymol., 1974, Vol. 34, W.B. Jakoby and M. Wilneck (Eds.), Academic Press: New York, NY; and M. Wilchek and E.A. Bayer, Anal. Biochem., 1988, 171: 1-32). Preferably, therapeutic entities are attached at positions on the antibody molecule or peptide molecule that do not interfere with the recognition properties of the resulting conjugate. In certain embodiments, the antibody molecule or peptide molecule is directly covalently linked to the therapeutic moiety. The direct covalent binding can be through a linkage such as a carbon-carbon, disulfide, amide, ester, carbamate, ether, thioether, urea, amino or carbonate linkage. Covalent binding can be achieved by taking advantage of functional groups present on the antibody/peptide and the therapeutic moiety. An activating agent, such as a carbodiimide, can be used to form a direct linkage. In other embodiments, the antibody or peptide molecule is covalently linked to the therapeutic moiety through a linker or spacer group. This can be accomplished by using any of a wide variety of stable bifunctional agents well known in the art, including homofunctional and heterofunctional linkers.

In certain embodiments, the TRPV2-binding peptide or antibody is conjugated to more than one therapeutic moiety, and/or to more than one type of therapeutic moiety.

As used herein, the term "therapeutic moiety" refers to a substance useful in the treatment, cure, prevention or suppression of a disease, disorder or medical condition, or in the treatment, cure, prevention or suppression of any symptoms associated with a disease, disorder or medical condition. Any of a wide variety of therapeutic moieties may be suitable for use in the practice of the present invention.

In certain embodiments, the therapeutic moiety is an anti-cancer agent, such as an anti-cancer agent belonging to one of the following classes of anti-cancer drugs: alkylating agents, purine antagonists, pyrimidine antagonists, plant alkaloids, intercalating antibiotics, aromatase inhibitors, anti-metabolites, mitotic inhibitors, growth factor inhibitors, cell cycle inhibitors, enzymes, topoisomerase inhibitors, biological response modifiers, anti-hormones and anti-androgens. In certain preferred embodiments, the anti-cancer drug is selected among anti-cancer drugs that are used for the treatment of melanoma, including, but not limited to temozolomide, interferon alfa-2b, aldesleukin, peginterferon alfa-2b, dacarbazine (also called DTIC), dabrafenib, Nabpaclitaxel, paclitaxel, carmustine (also called BCNU), cisplatin, carboplatin, vinblastine, Vemurafenib (Zelboraf) and dabrafenib (Tafinlar), trametinib (Mekinist), imatinib (Gleevec) and nilotinib (Tasigna).

The therapeutic moiety may be a nano-metallic cluster *(e.g.,* gold nano-particles, nano-spheres, nano-tubes or other nano-constructs) which, when irradiated with electromagnetic radiation, kill cells in the vicinity of the metallic cluster; or a boron cluster (e.g., closo-boron, a cluster containing 12 boron atoms) which, when irradiated with slow thermal neutrons, produce energetic alpha particles that kill nearby cells.

The therapeutic moiety may also be selected among a large variety of biologically active compounds that are known to have a beneficial effect to a patient in general *(e.g.,* anti-inflammatory agents, immunomodulatory agents, analgesics, antimicrobial agents, antibacterial agents, antibiotics, antioxidants, antiseptic agents, and combinations thereof).

### B. Indications

As indicated above, the TRPV2 agonists and TRPV2-binding compounds conjugated to therapeutic moieties, as described above, may be used in the treatment of melanoma, whereas the TRPV2 blockers or inhibitors *(i.e.,* TRPV2 antagonists and RNAi agents) may be used in the prevention or inhibition of melanoma metastasis formation.

Methods of treatment or prevention of the present invention may be accomplished using a TRPV2 therapeutic agent, or a pharmaceutical composition thereof. These methods generally comprise administration of a therapeutically effective amount of a TRPV2 therapeutic agent, or a pharmaceutical composition thereof, to a subject in need thereof. Administration may be performed using any of the methods known to one skilled in the art. In general, the TRPV2 therapeutic agent, or a pharmaceutical composition thereof, will be administered in an effective amount, *i.e.,* an amount that is sufficient to fulfill its intended purpose. The exact amount of the TRPV2 therapeutic agent to be administered will vary from subject to subject, depending on the age, sex, weight and general health condition of the subject to be treated, the desired biological or medical response and the like. The effects of a treatment according to the invention may be monitored using any of the diagnostic assays, tests and procedures known in the art.

In certain embodiments, the TRPV2 therapeutic agent, or a pharmaceutical composition thereof, is administered alone. In other embodiments, the TRPV2 therapeutic agent, or a pharmaceutical composition thereof, is administered in combination with at least one additional therapeutic agent or therapeutic procedure. The TRPV2 therapeutic agent, or pharmaceutical composition thereof, may be administered prior to administration of the therapeutic agent or therapeutic procedure, concurrently with the therapeutic agent or procedure, and/or following administration of the therapeutic agent or procedure.

Therapeutic agents that may be administered in combination with the TRPV2 therapeutic agent, or a pharmaceutical composition thereof, may be selected among a large variety of biologically active compounds that are known to have a beneficial effect in the treatment of cancer (in particular melanoma) or to a patient in general *(e.g.* anti-cancer agents, anti-inflammatory agents, immunomodulatory agents, analgesics, antimicrobial agents, antibacterial agents, antibiotics, antioxidants, antiseptic agents, and combinations thereof). In certain embodiments, the TRPV2 therapeutic agent is administered in combination with at least one of: temozolomide, interferon alfa-2b, aldesleukin, peginterferon alfa-2b, dacarbazine, dabrafenib, nivolumab, penbrolizumab, ipilimumab, and trametinib, vemurafenib, and any combination thereof.

Therapeutic procedure that may be administered in combination with the TRPV2 therapeutic agent, or a pharmaceutical composition thereof, may be selected among surgery and radiotherapy.

### C. Administration

The TRPV2 therapeutic agents as described above (optionally after formulation with one or more appropriate pharmaceutically acceptable carriers or excipients), in a desired dosage can be administered to a subject in need thereof by any suitable route. Various delivery systems are known and can be used to administer a TRPV2 therapeutic agent of the present invention, including tablets, capsules, injectable solutions, encapsulation in liposomes, microparticles, microcapsules, etc. Methods of administration include, but are not limited to, dermal, intradermal, intramuscular, intraperitoneal, intralesional, intravenous, subcutaneous, intranasal, pulmonary, epidural, ocular, and oral routes. A TRPV2 therapeutic agent, or a composition thereof, may be administered by any convenient or other appropriate route, for example, by infusion or bolus injection, by adsorption through epithelial or mucocutaneous linings (e.g., oral, mucosa, rectal and intestinal mucosa, etc). Administration can be systemic or local. Parenteral administration may be directed to a given tissue of the patient, such as by catheterization. As will be appreciated by those of ordinary skill in the art, in embodiments where the TRPV2 therapeutic agent is administered along with an additional therapeutic agent, the TRPV2 therapeutic agent and the therapeutic agent may be administered by the same route *(e.g.,* orally) or by different routes *(e.g.,* orally and intravenously).

Administration of a TRPV2 therapeutic agent (or a pharmaceutical composition thereof) according to the present invention will be in a dosage such that the amount delivered is effective for the intended purpose. The route of administration, formulation and dosage administered will depend upon the therapeutic effect desired, the severity of the disorder being treated, the presence of any infection, the age, sex, weight and general health condition of the patient as well as upon the potency, bioavailability and *in vivo* half-life of the TRPV2 therapeutic agent, the use (or not) of concomitant therapies, and other clinical factors. These factors are readily determinable by the attending physician in the course of the therapy. Alternatively or additionally, the dosage to be administered can be determined from studies using animal models. Adjusting the dose to achieve maximal efficacy based on these or other methods is well known in the art and is within the capabilities of trained physicians.

A treatment according to the present invention may consist of a single dose or multiple doses. Thus, administration of a TRPV2 therapeutic agent, or a composition thereof, may be constant for a certain period of time or periodic and at specific intervals, *e.g.,* hourly, daily, weekly (or at some other multiple day interval); monthly, yearly *(e.g.,* in a time release form). Alternatively, the delivery may occur at multiple times during a given time period, *e.g*., two or more times per week, two or more times per month, and the like. The delivery may be continuous delivery for a period of time, *e.g*., intravenous delivery.

### D. Pharmaceutical Compositions

As mentioned above, a TRPV2 therapeutic agent of the invention may be administered *per se* or as a pharmaceutical composition. Accordingly, the present invention provides pharmaceutical compositions comprising an effective amount of a TRPV2 therapeutic agent and at least one pharmaceutically acceptable carrier or excipient. In some embodiments, the composition further comprises one or more additional biologically active agents.

The pharmaceutical compositions of the present invention may be formulated in dosage unit form for ease of administration and uniformity of dosage. The expression "unit dosage form", as used herein, refers to a physically discrete unit of for the patient to be treated. It will be understood, however, that the total daily dosage of the compositions will be decided by the attending physician within the scope of sound medical judgement.

### Formulation

It is within the capabilities of one skilled in the art to select and use pharmaceutically acceptable media, carriers and excipients to formulate pharmaceutically active substances (see for example "Remington's Pharmaceutical Sciences", E.W. Martin, 18th Ed., 1990, Mack Publishing Co.: Easton, PA, which is incorporated herein by reference in its entirety). The TRPV2 therapeutic agents may be formulated into injectable preparations, depot injectable preparations, liquid dosage forms, solid dosage forms, topical formulations, and the like.

### Additional Biologically Active Agents

In certain embodiments, the TRPV2 therapeutic agent is the only active ingredient in a pharmaceutical composition of the present invention. In other embodiments, the pharmaceutical composition further comprises one or more biologically active agents. Examples of suitable biologically active agents include, but are not limited to, anti-cancer agents, anti-inflammatory agents, immunomodulatory agents, analgesics, antimicrobial agents, antibacterial agents, antibiotics, antioxidants, antiseptic agents, and combinations thereof. Examples of specific anti-cancer agents used in the treatment of melanoma have been listed above.

In such pharmaceutical compositions, the TRPV2 therapeutic agent and the at least one additional biologically active agent may be combined in one or more preparations for simultaneous, separate or sequential administration of the TRPV2 therapeutic agent and biologically active agent(s). More specifically, an inventive composition may be formulated in such a way that the TRPV2 therapeutic agent and biologically active agent(s) can be administered together or independently from each other. For example, the TRPV2 inhibitor and a biologically active agent can be formulated together in a single composition. Alternatively, they may be maintained (*e.g*., in different compositions and/or containers) and administered separately.

Further aspects and advantages of this invention will be disclosed in the following figures and examples, which should be regarded as illustrative and not limiting the scope of this application.

### Brief Description of the Drawing

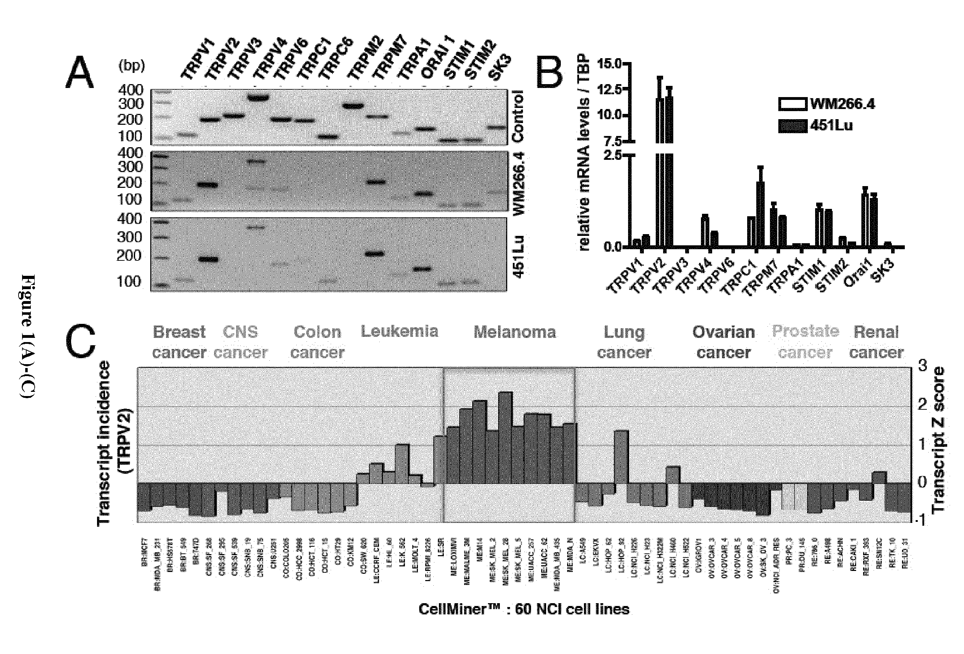

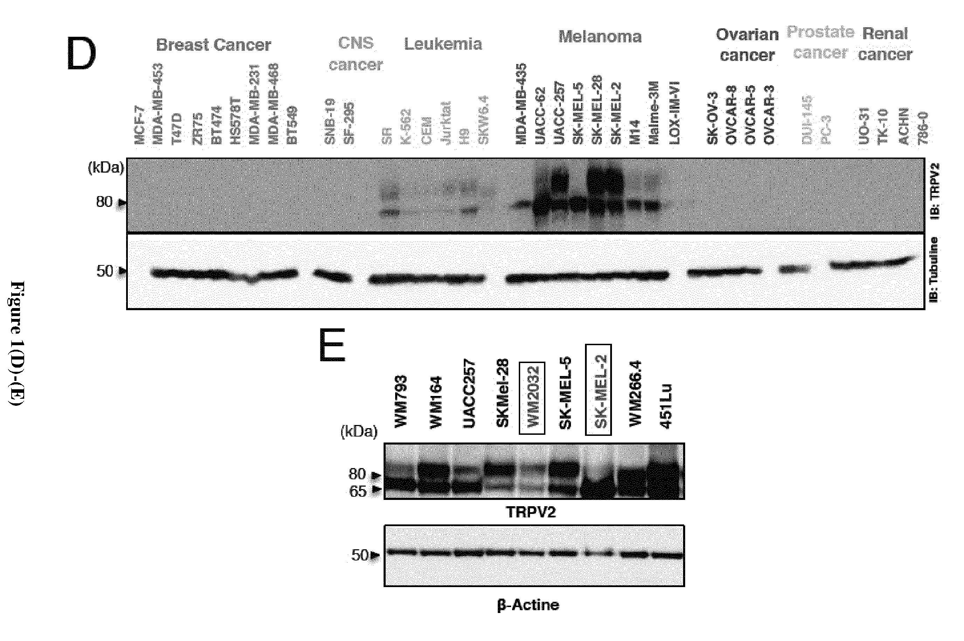

**Figure 1****. TRPV2 is highly overexpressed in melanoma cells. (A)** Expression profile of different ion channels analyzed by RT-PCR in the metastatic melanoma cell lines; WM266.4 (middle panel) and 451Lu (bottom panel) compared to expected amplicons (top panel). The amplicons sizes are expressed in bp: TRPV1=120 bp, TRPV2=199 b, TRPV3=226 bp, TRPV4=190/370 bp, TRPV6=208 bp, TRPVc=201 bp, TRPC6=121 bp, TRPM2=303 bp, TRPM7=226 bp, TRPA1=269 bp, Orai1=161 bp, and SK3=174 bp). **(B)** Quantitative RT-PCR analyses of ion channel expression in WM266.4 and 451Lu melanoma cell lines normalized to TATA box-binding protein (TBP). **(C)** CELLMINER™ relative TRPV2 mRNA expression analysis in the NCI-60 cell lines panel (website: discover.nci.nih.gov/cellminer/home.do). **(D)** Western blot analysis of 40 NCI cell lines showing TRPV2 expression. Actin was used as a loading control. **(E)** Western blot analysis of TRPV2 expression in melanoma model cell lines harboring the B-RAF (non-framed) or N-Ras (framed) mutation.

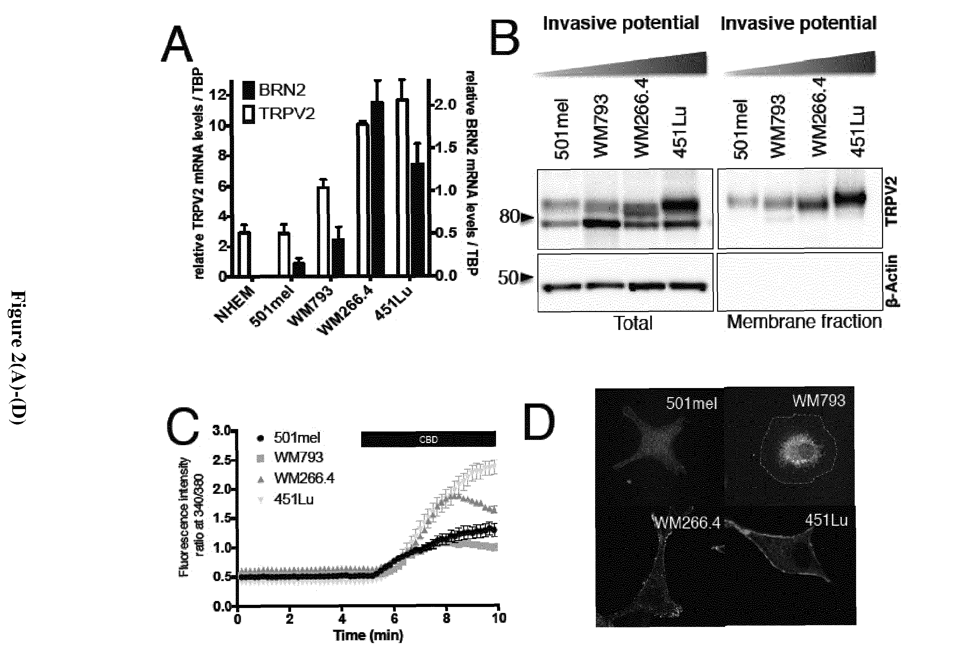

**Figure 2****. TRPV2 expression and function correlate with the invasive potential of melanoma cell lines *in vitro.* (A)** Quantitative RT-PCR of TRPV2 expression and of BRN2 expression in the non-invasive 501mel, VGP WM793, skin metastasis WM266.4 and the highly metastatic xenograft-derived 451Lu. BRN2 was used as a marker of the melanoma invasive phenotype. **(B)** Western-blot analyses of TRPV2 total and plasma membrane (PM) expression in the non-invasive 501meln VGP WM793, skin metastasis WM266.4 and the highly metastatic xenograph-derived 451Lu. Anti-β-Actin was used as loading and cell integrity control. PM fractions were isolated using the surface protein biotinylation technique. **(C)** Confocal microphotographs showing the distribution of TRPV2 in melanoma cell lines. Doted line highlights membrane edge in the MW793 cell line. **(D)** Representative Ca2+ influx response to cannabidiol (CBD) indicated by the bar. Data are presented as mean ± SD from at least 20 cells.

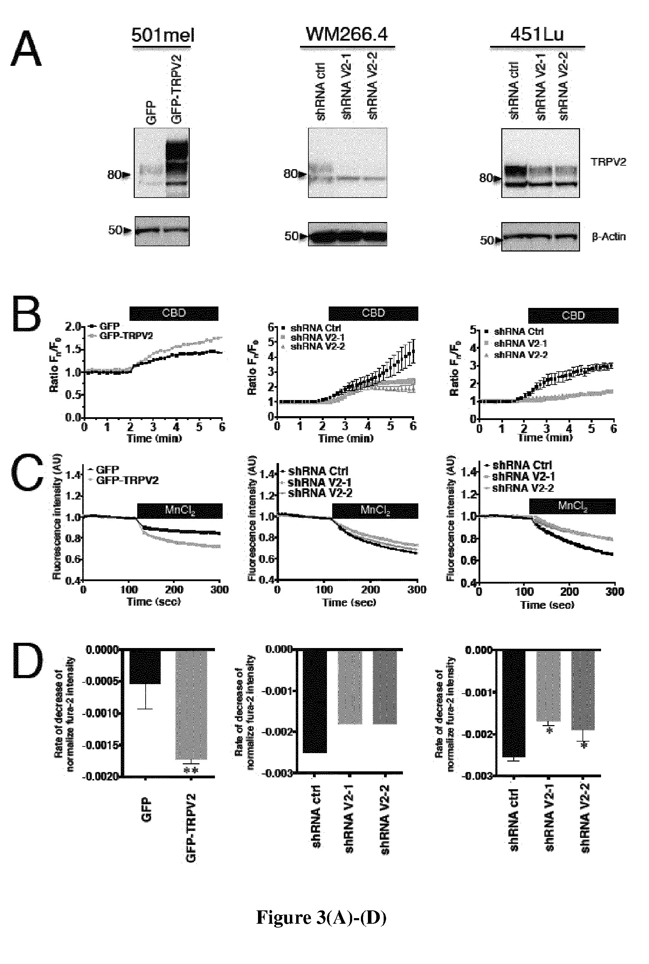

**Figure 3****. TRPV2 participates in the basal calcium entry response in melanoma. (A)** TRPV2 over-expression in the non-invasive 501mel cell line and downregulation by lentiviral-delivery of specific shRNA in the WM266.4 and 451Lu metastatic melanoma cell lines verified by western-blot (top panels). Actin was used as a loading control (bottom panels). **(B)** Representative kinetics of CBD induced Ca2+ influx in 501mel-GFP and 501mel-TRPV2 GFP (left panel), WM266.4 shRNA Ctrl, shRNA1 TRPV2 and shRNA2 TRPV2 (middle panel) and 451Lu shRNA Ctrl, shRNA1 TRPV2 and shRNA2 TRPV2 (right panel). **(C)** Assessment of basal calcium permeability using Mn2+ quenching of Fura-2 loaded in control or TRPV2 overexpressed 501mel cells (left panel), or WM266.4 and 451Lu control or expressing 2 specific shRNA TRPV2 messengers. **(D)** Average normalized quenching rates of 501mel cells, WM266.4 and 451Lu transfectants. Color code of the bar charts for downregulated cell lines: black (shRNA control), light grey (shRNA1 TRPV2). Statistical significance was obtained by an ANOVA test.

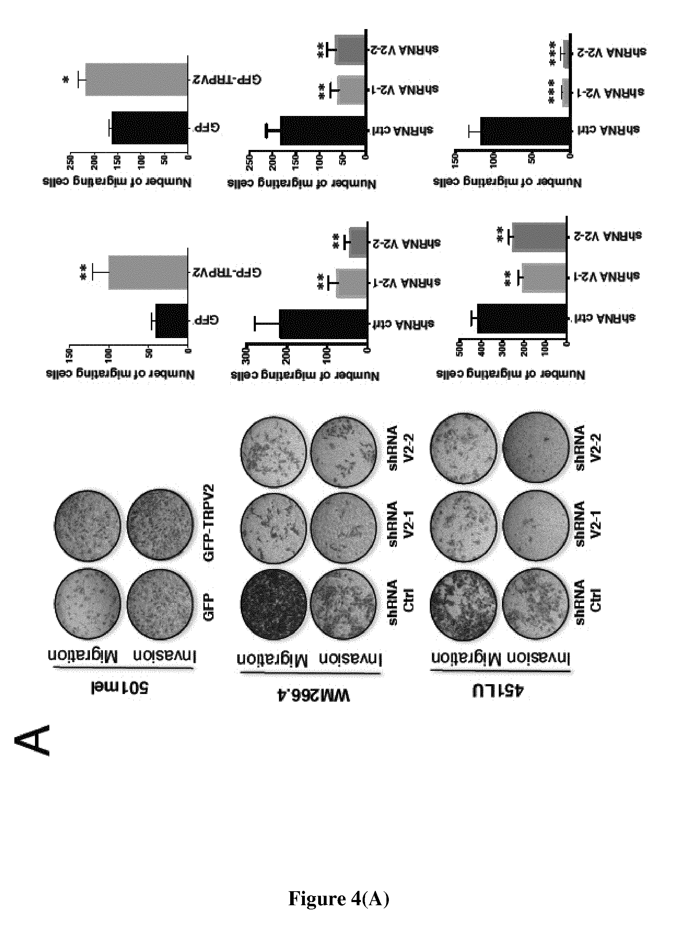

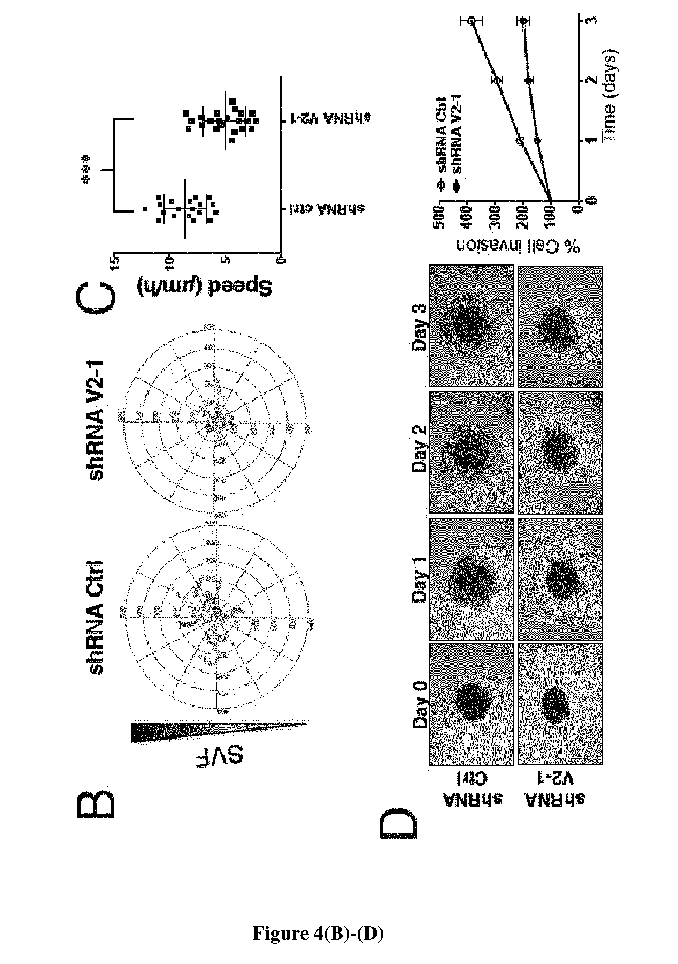

**Figure 4****. Overexpression or knockdown of TRPV2 modulates cell migration. (A)** Impact of TRPV2 genetic manipulation on serum-induced in 501mel (top), WM266.4 (middle) and 451Lu (bottom) migration and Matrigel invasion (assessed in Boyden chambers experiments). The pictures represent stained cells that have migrated through 8µm pore size membranes. Histograms represent the average of the number of migrating cells from three (3) independent experiments, error bars, SE. **(B)** Tracks comparison between WM266.4 shRNA Ctrl or shRNA1 TRPV2 cells migrating toward an FCS gradient over a 12 hour period (n = each). The left panel shows the comparison of velocity. **(C)** TRPV2 downregulation effect on 3D invasion of collagen-embedded WM266.4 spheroids. The left panel shows means ± SEM of three (3) independent experiments.

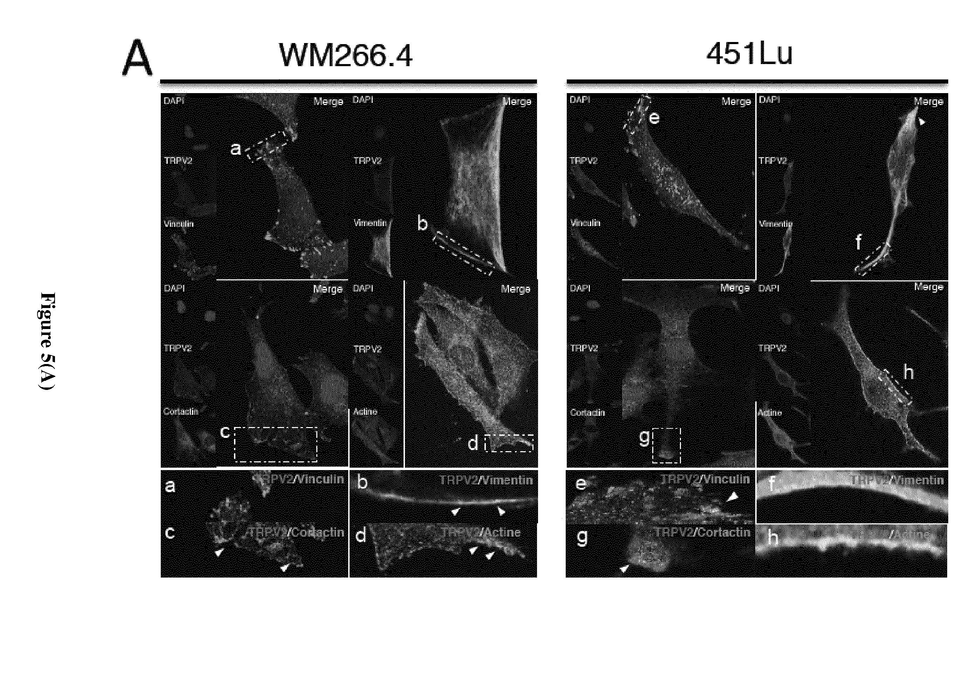

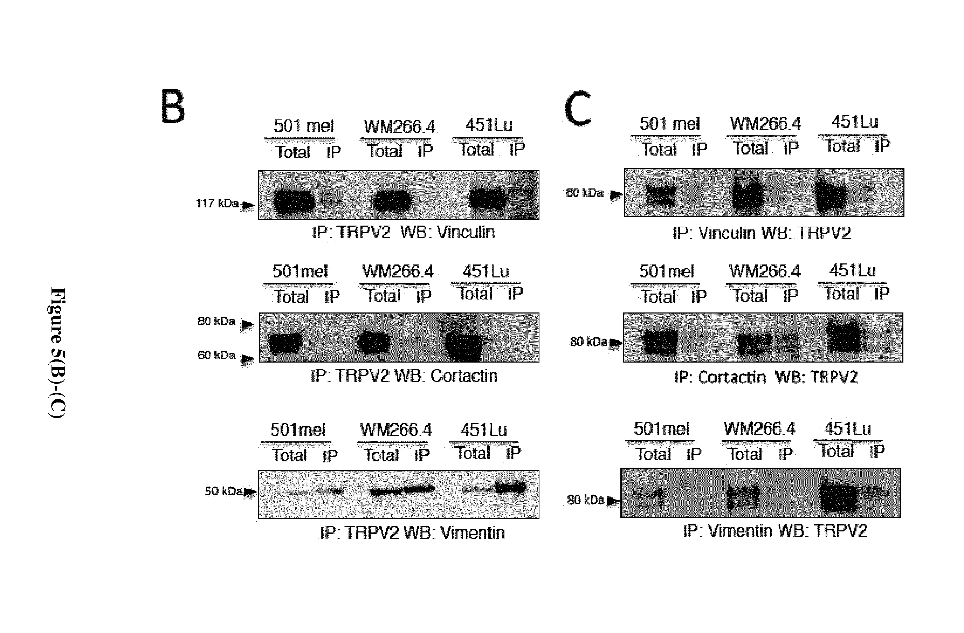

**Figure 5****. TRPV2 channels interact with the focal adhesion and cell migration machinery. (A)** Representative confocal images of low confluence melanoma cell lines WM266.4 and 451Lu seeded on fibronectin coated coverslips. Cell nuclei depicted in blue, TRPV2 in red and indicated protein in green. Insets: magnification of the indicated area of panels (bottom panels). The arrows indicate the sites of co-localization. **(B)** Co-immunoprecipitation of Vinculin, Cortactin, and Vimentin from melanoma cell lines 501mel, WM266.4 and 451Lu using an anti-TRPV2 antibody (left panels). **(C)** Reverse co-immunoprecipitation showing TRPV2 pulldown from melanoma cell lines 501mel, WM266.4 and 451Lu using a Vinculin (top), Cortactin (middle), or Vimentin (bottom) antibody. All co-immunoprecipitation were performed three (3) times and the Western blots show typical results. Anti-GFP antibody was used as an isotype control antibody.

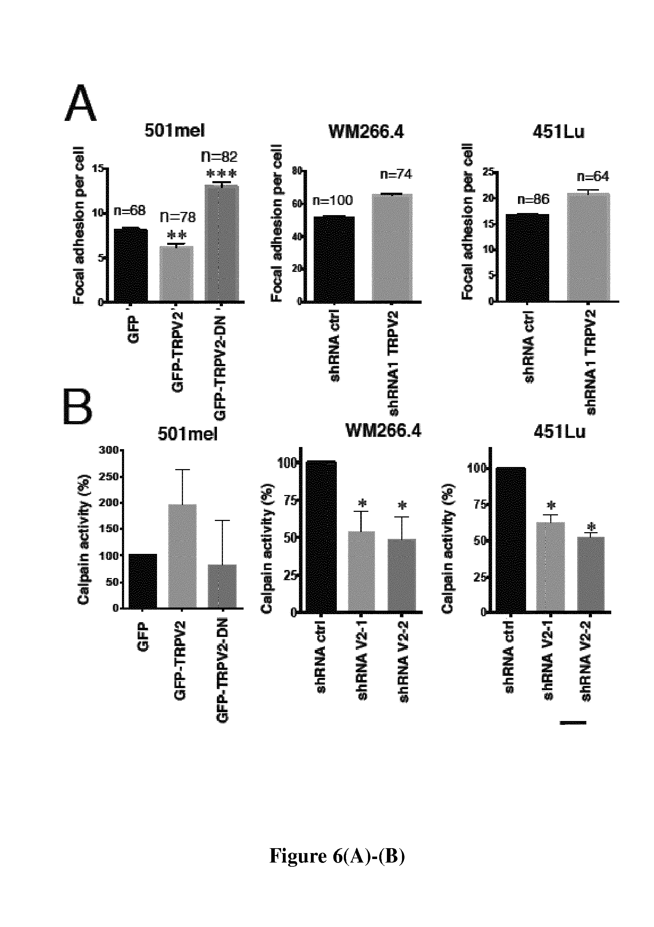

**Figure 6****. TRPV2 activity modified focal adhesion dynamics and actin polymerization. (A)** Quantification of focal adhesion points per cells analyzed by vinculin staining. Images were analyzed using the imageJ software. The number on top represents the number of cells counted on each cell line. **(B)** Histograms represent the average percentage of basal calpain activity in 501mel cells control, overexpressing TRPV2 or overexpressing a dominant negative form of TRPV2 (left panel) or in control, shRNA1 or shRNA2 TRPV2 cells WM266.4 (middle panel) or 451Lu (right panel). Bar graphs show means ± SEM (n=3 independent experiments.

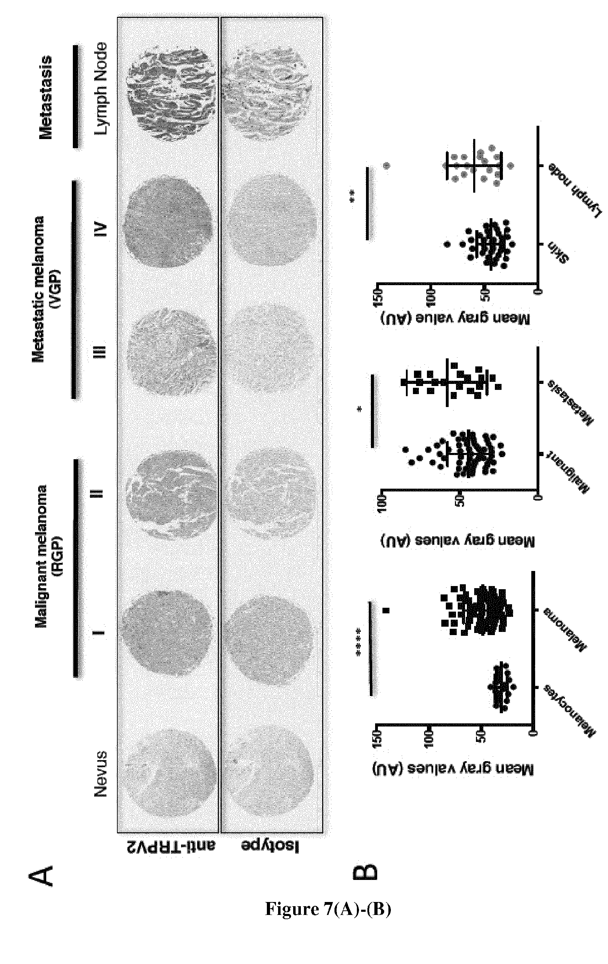

**Figure 7****. TRPV2 is over-expressed in human melanoma samples. (A)** Representative melanoma tissue samples from tissue microarray (TMA), TRPV2 staining (top panel) and isotype control (bottom panel) from nevus, malignant melanoma (grades I-IV) and metastatic melanoma samples. Scale bar: 450 nm. **(B)** Quantitation showing TRPV2 staining from TMA samples in 62 patients with malignant melanoma, 20 patients with metastatic malignant melanoma and 18 nevi. Analysis was performed using the imageJ software (NIH, Bethesda, USA). Values represent mean grey value ± SD. Statistical analysis using unpaired t test, * p<0.01, ** p<.0005, **** p<0.0001.

### Examples

The following examples describe some of the preferred modes of making and practicing the present invention. However, it should be understood that the examples are for illustrative purposes only and are not meant to limit the scope of the invention. Furthermore, unless the description in an Example is presented in the past tense, the text, like the rest of the specification, is not intended to suggest that experiments were actually performed or data were actually obtained.

### Materials and Methods

### Cells

The melanoma cell lines: WM793, 501MEL, 451Lu, WM164, UACC162, UACC257, SK-MEL2, SK-MEL5, SK-MEL28, MDA-MB435, MALME-3M, LOX-IM-VI were maintained in DMEM supplemented with 8% v/v heat-inactivated FCS and 2 mM L-Glutamine. The WM266.4 cell line was maintained in RPMI1640 supplemented with 8% v/v heat-inactivated FCS. Cells from the WM2032 cell line were maintained in TU medium supplemented with 2% heat-inactivated FCS.

Cells from the NCI-60 collection of human tumor cell lines came from Charles River Laboratories (Wilmington, MA, USA), WM266.4 were purchased at ATCC. WM164, WM2032 and 451LU come from the WISTAR institute. Human 501mel cells were kindly supplied by Sophie Tartare-Deckert, Inserm U1065, Nice.

For transfection, cells were electroporated with 10 µg of DNA using the BTM 830 electroporation generator (BTX Instrument Division, Harvard Apparatus). For selection of stable clones, transfected/transduced cells were placed in a medium supplemented with selective doses of Zeocine or puromycin antibiotics.

### shRNA Production and Transfection

Validated non-silencing control and TRPV2 targeting shRNAmir-pGIPZ lentiviral vectors were purchased from Dharmacon. Lentiviral particles and cell transduction were performed according to manufacturer instructions.

### Antibodies and Reagents

The antibodies that were used in this study are: anti actin (Sigma A5316), anti VRL-1 (H-105, Santa Cruz Biotechnologie), anti-Vimentin (Clone V9, M0725 DAKO), anti-Cortactin (Millipore), anti-Vinculin (Clone VLN01, MA5-11690 Life technologies), Rhodamine-Phalloidine (Molecular probe) and SIR-actin (Spirochrome).

### Plasmids and Constructs

The eGFP coding sequence from the pEGFP-C2 vector (Clontech) with or without the wild-type and dominant-negative mutant human TRPV2 coding sequence (Penna et al., Cell Calcium 2006, 39, 495-507; Juvin et al. Molecular Pharmacology 2007, 72, :1258-1268) added in 3' were inserted into the pcDNA3.1(+)-Zeocine vector (Life Technologies) by endonuclease restriction digestion and ligation. All constructs were verified by DNA sequencing.

### Cell Miner Metadata

Data was generated querying TRPV2 as input in Cellminer (Website: discover.nci.nih.gov/cellminer/) as described in Shankavaram et al., BMC Genomics, 2009, 10: 277 and Reinhold et al., Cancer Res., 2012, 72(14): 3499-24511.

### RNA Extraction, Reverse-Transcription and PCR

Total RNA was extracted from cell samples using a Nucleospin RAII kit (Macherey-Nagel) and following the manufacturer's instructions. Recovered RNA samples were quantified using a NanoDrop spectrophotometer ND1000 (Thermo Fisher Scientific). Reverse transcription was performed on 0.5 µg of total RNA in a volume of 20 µL using a High capacity cDNA Reverse Transcription kit (Applied Biosystems) according to the manufacturer's instructions. Quantitative PCR was performed on 0.5 ng cDNA samples, in sealed 96-well microtiter plates using the SYBR GreenTM PCR Master Mix (Applied Biosystems) with the 7300 SDS Real-Time PCR System (Applied Biosystems). Transcript relative amounts were determined using the deltadelta-Ct method and human TBP transcript level was used as internal normalizer for each sample.

### Biochemical Techniques

Western-blots of total cell lysates were performed as previously described (Edmond et al., Oncogene, 2015, 34: 996-1005). Briefly, the cells were washed once with ice cold PBS and permeabilized using RIPA buffer supplemented with a cocktail of protease and phosphatase inhibitors. Protein concentration was determined using the bicinchoninic acid method (Pierce, Rockford, IL, USA) according to the manufacturer's protocol. A total of 30 µg of protein was loaded per lane, and the protein samples were later resolved using a 4-12% SDS-PAGE gel (NuPage) and transferred to a nitrocellulose membrane (GE Healthcare, Buckinghamshire, UK) according to the manufacturer's protocol. Non-specific binding sites were blocked by incubating the membranes for 30 minutes with TBST-BSA (50 mM Tris, 160 mM NaCl, 0.05% (v/v) Tween 20, and 5% BSA at pH 7.4) or TBST-milk (50 mM Tris, 160 mM NaCl, 0.05% (v/v) Tween 20 and 5% (w/v) dried skimmed milk at pH 7.4) and later incubated overnight at 4°C with the appropriate primary antibody. For primary antibody visualization, the membranes were washed with TBST, incubated with an appropriate peroxidase-labeled antibody for 45 minutes and then visualized using an enhanced chemiluminescence substrate kit (ECL RevelBot Ozyme, Saint Quentin en Yvelines, France) according to the protocols provided by the manufacturer.

Co-immunoprecipitation was performed using cells cultured in 10 mm fibronectin-coated dishes. After sample preparation, 1.5 mg of cell lysate per sample was used for immunoprecipitation as described elsewhere (Penna et al., Nature 2008, 456: 116-120). Briefly, supernatants were tumbled for 20 minutes on ice followed by 3 hours of incubation at 4°C with protein A-Sepharose beads (Sigma Aldrich) with the appropriate primary antibody. The beads were then washed five times with a lysis buffer, resuspended in Laemmli sample buffer and boiled for 5 minutes. The samples were resolved and blotted according to the above-described protocol.

The biotinylation assays were carried out using Sulfo-NHS-SS-Biotin (Pierce Biotechnology) according to the manufacturer's instructions. Briefly, cells were cultured to approximately 75% confluence in 6-well plate dishes and serum-deprived for 12 hours. They were then washed three times in cold PBS and incubated in the presence of the biotinylation reagent (Sulfo-Link NHS-LC-biotin, Pierce, 0.5 mg/mL in PBS pH 8). Following a 30 minute incubation period, the biotin reagent was removed and the cells were washed twice in PBS containing 100 mM of glycine and twice in PBS alone. Next, the lysates were prepared as described above and incubated with 50 µl of streptavidin-agarose beads (Invitrogen) for 1 hour at 4°C. The beads were then pelleted and washed three times in lysis buffer. Captured proteins were eluted with Laemmli sample buffer, boiled at 95°C for 5 minutes, and then analyzed by western blot with appropriate antibodies.

### Immunohistochemistry

The melanoma tissue microarray ME1004c (US Biomax Inc., Rockville, MD) composed of 62 patients with malignant melanoma, 20 patients with metastatic malignant melanoma and 18 nevus was used to assess TRPV2 expression analysis. Immunohistochemical staining was performed using a Discovery Automated IHC stainer (Roche^{®}, Illkirsh, France). Slides were stained using an anti-TRPV2 antibody at a dilution of (1:2000) for 60 minutes at 37°C. Signal enhancement was performed using the Ventana ChromoMap Kit Slides (biotin free detection system). Sections were counterstained with hematoxylin and mounted with DPX mounting medium. Histological information including TNM, clinical stage and pathology grade, with IHC results of HMB45 were obtained with the array.

### Fluorescence and Confocal Microscopy

Immunofluorescence was performed in melanoma cells seeded in low confluence on glass cover slips coated with fibronectin for 24 hours. Next, the cells were washed twice with PBS and then fixed with 4% PFA or 70% cold ethanol. After being washed a second time, the cells were incubated for 20 minutes in a blocking solution (PBS+1% heat-inactivated FCS). First, the primary antibody was incubated for 20 minutes at 4°C and then visualized using either a secondary mouse or rabbit antibody coupled to Alexa fluor 555 (AF555) or coupled to Alexa fluor 488 (AF488). Fluorescence micrographs were obtained using a laser scanning confocal system (TCS SP8 model mounted on a DMI 6000 CS inverted microscope, Leica, Heidelberg, Germany). The images were taken using a 40x oil-immersion objective (1.4 N.A.), collected in a format of 1,024 x 1,024 pixels and analyzed using the ImageJ software (NIH, Bethesda, Maryland, USA).

### Intracellular Ca²⁺ Imaging

Cells plated on glass coverslips were loaded with 5 µM Fura-2-AM in DMEM at 37°C for 45 minutes and then washed three times in HBSS solution (142.6 mM NaCl, 5.6 mM KCl, 2 mM CaCl2, 1 mM MgCl₂, 10 mM HEPES, 5 mM Glucose, pH 7.4) followed by de-esterification at 37°C for 15 minutes. The experimental protocol for [Ca²⁺]i imaging involved data acquisition every 10 seconds at wavelengths of 340 nm and 380nm, using a LEICA DMIRB equipped with a 40x /1.35 UApo N340 (Olympus) inverted objective. Changes were monitored using an imaging system equipped with a CoolSnapHQ fast-cooled monochromatic digital camera (12-bit) (Princeton Instrument), a monochromator for fluorophore excitation, and the METAFLUOR software (Universal Imaging, Downingtown, PA, USA) for image acquisition and analysis. All the images were background-subtracted. The cells with spontaneous or aberrant Ca²⁺ activity were identified by imaging and eliminated from the analysis. Depicted curves represent a minimum of three (3) independent experiments.

### Measurement of Basal Calcium Permeability by Mn²⁺ Quenching in Fura 2-Loaded Cells

Basal calcium permeability was measured in melanoma cells seeded in black glass-bottom 96-well plates. A baseline was established using a HBSS solution. Two hundred (200) nanomolar MnCl₂ was added at the indicated time point. Mn²⁺ entry was measured after the rate of decline (quenching) of fura-2 intensity measured at its isosbestic wavelength, 359 nm. The measures were acquired every 3 seconds. Each point represents the mean of quadruplicates normalized to the baseline.

### In vitro Migration/Invasion Assays

Migration and invasion experiments were performed using 8 µm pore Boyden chambers (Millipore, Molsheim, France). After 30 minutes of membrane hydration, 2x10⁵ cells of the 501MEL or WM266.4 cell line or 4x10⁵cells of the 451Lu cell line were added to the top chamber. Cell chemo-attraction was induced by adding 10% of fetal calf serum to the bottom chamber. After 12 hours of incubation, the cells were fixed in 70% cold ethanol and stained with Crystal Violet. The stained cells were then removed from the top-side of the membrane using a cotton-tipped swab and five representative pictures for each insert were taken and the cells counted using cell counter (ImageJ).

### 2D Cell Migration and Cell Tracking

Cell tracking was performed using cells of the WM266.4 cell line that were starved for 12 hours and that expressed control shRNA or TRPV2 shRNA. The cells were seeded in a 2D migration chamber coated with fibronectin (Ibidi, Biovalley, CA) filled with serum-free RPMI medium. Migration was carried out for 12 hours at 37°C and 5% CO₂ throughout the experiment. Migration was induced by adding 5 µl of FCS in the upper chamber. The experiments were performed using an inverted microscope (Olympus IX71) equipped with a motorized stage. Traces and migration speed were analyzed using NIH ImageJ analysis software.

### Three-Dimensional Spheroid Growth

Melanoma spheroids were generated using the liquid overlay technique and implanted into a type I collagen matrix in growth medium as previously described (Baillet et al., Cancer Res, 2009, 69: 2748-2756). Tumor cell outgrowth was visualized by phase contrast microscopy and the radius of cell growth was analyzed by ImageJ.

### Cell Proliferation

Cell viability was evaluated using the MTT assay, as previously described (Ning et al., Mol. Cancer Res., 2007, 12: 1254-62). Briefly, forty thousand (40,000) cells were cultured for 24 hours in flat-bottom 96-well plates in the presence of an appropriate concentration of the apoptosis inducer in a final volume of 100 µl. Then, 15 µl of MTT (5 mg/ml in PBS) solution were added, and after 4 hours of incubation at 37°C, the absorbance was measured at 570 nm using the EnSpire® 2300 Multilabel Plate Reader reader (Perkin Elmer, Waltham, MA, USA). Proliferation was also assessed by cell counting. Briefly, the cells were cultured in 6-well dishes in duplicate at a density of 5,000 or 20,000 cells per well in 2 ml of medium supplemented with or without FBS. The medium was changed every 48 hours. Cell number at the indicated time points was determined by counting using a haemocytometer.

### Substrate Zymography

The serum free media from 451Lu, 501mel, or WM266.4 transfected cells was collected after the indicated times of stimulation and loaded on a 8% SDS-PAGE containing 1 mg/ml type I collagen. The protein concentration was quantified by BCA (bicinchoninic acid assay). Following electrophoresis, the proteins were renatured by incubating gels in 2.5% Triton X-100 for 1 hour at room temperature. The gels were then washed 3 times in distilled water and incubated in substrate buffer for 12 hours at 37°C. Finally, the gels were revealed in a stain buffer (0.1% Coomassie Blue, 10% acetic acid) for 15 minutes at room temperature, and washed twice with a 7% acetic acid solution.

### Calpain Activity

Calpain activity was measured using the Calpain activity assay kit (Promokine, #PK-CA577-K240, Germany). Briefly, 2x10⁶ cells were seeded on 6-well plates for 12 hours. Then, the cell samples were washed twice in PBS and the cells were resuspended in 100 µl of extraction buffer. After protein measurement, 200 µg of protein of sample were diluted in 85 µl of extraction buffer. Calpain activity was measured by adding 10 µl of the reaction buffer and 5 µl of fluorescent calpain substrate. The calpain activity was then measured using a fluorometer equipped with a 400 nm excitation filter and a 505 nm emission filter. In each case, 1 µl of calpain inhibitor was added to subtract the sample background.

### Zebrafish Tumor Cell Implantation and Micrometastasis Analysis

Zebrafish embryos were raised, staged, and maintained according to standard procedure. For cell microinjection, 2 days-post fertilisation (dpf), phenylthiourea (PTU, Sigma-Aldrich)-treated zebrafishes were dechorionized and tricaine anesthetized. The cells were labelled using the CM-Dil tracker, mixed at equal quantity and loaded into borosilicate capillaries at a density of 3x10⁶ cells/ml. Injections were performed using a pneumatic picopump (World Precision Instruments) and a micromanipulator. For all experiments, cell injection was performed above the ventral duct of Cuvie (DoC), as previously described (Teng et al., BMC Cancer, 2013, 13: 453). After confirmation of a visible cell mass at the injection site, zebrafishes were transferred to an incubator and maintained at 34°C for 36 hours.

Micrometastasis formation was analyzed on living zebrafish embryos anesthetized using Tricaine. Images were captured using a DeltaVision imaging system and processed using ImageJ/Fiji software.

### Statistical Analysis

All data are presented as mean values from at least 3 independent experiments. Statistical differences among cell lines or treatments were performed using an ANOVA test for multiple comparisons, or using the paired Student *t* test, as appropriate using the Prism 6.0 (graphPad software). Values with a P value < 0.05 were considered statistically significant.

### Results

### TRPV2 is highly overexpressed in both B-RAF and N-Ras melanoma cells

To identify the Ca²⁺ channel subunits contributing to melanoma metastasis formation, the present Inventors screened, by RT-PCR, a set of invasive melanoma cell lines for expression of a panel of non-voltage gated Ca²⁺ -channels selected for their ability to promote the metastatic behavior of some cancer cells (Nielsen et al., Br. J. Pharmacol., 2014, 171: 5524-5540). Among these Ca²⁺-channels, TRP-V1, TRPV2, TRPV4, TRPV6, TRPC1, TRPM7, TRPA1 and Orai1 mRNAs were found consistently expressed in every cell lines tested (Figure 1A and data not shown). Quantification of their mRNA levels by RT-PCR revealed that TRPV2 was the subunit predominantly expressed, the amount of TRPV2 transcripts present exceeding by far the expression of the other channel analyzed (Figure 1B). Interestingly, query of the transcriptomics data available for the NCI-60 panel (Reinhold et al., Cancer Res, 2012, 72: 3499-3511) further showed that this strikingly high expression is a melanoma particular feature (Figure 1C). This last observation was corroborated at the protein level, in a subset of NCI-60 panel cell lines from cancers originating from different tissues (Figure 1D). More than 50% of melanomas harbor BRAF mutations, which can be targeted with BRAF inhibitors, while another 20% have *NRAS* mutations. These 'driver mutations' are mutually exclusive, but both lead to the constitutive activation of the MAPK signaling pathway causing uncontrolled proliferation and apoptosis resistance. Thus, the present Inventors decided to analyze TRPV2 protein expression in an extended set of model melanoma cell lines harboring either B-RAF or N-Ras gene mutations. Noteworthy, TRPV2 high expression levels were consistently observed, irrespective of the mutational status of the cell line (Figure 1E). Considering TRPV2 predominant and ubiquitous expression in melanoma, the present Inventors decided to specifically focus their attention on this channel subunit.

### TRPV2 expression levels correlate with the invasive phenotype of the tumor

Typically, during melanomagenesis, primary lesions progress to malignant tumors through a multistep process. First melanocytes proliferate, forming dysplasia. Following this radial growth phase (RGP), tumor cells start to invade the surrounding tissue through a vertical growth phase (VGP) and ultimately reach the circulation and spread to distant sites. Once finding a suitable environment in another tissue, cells colonize this tissue by reverting to a more proliferative phenotype. Thus, melanoma cells have the ability to go back and forth between proliferative and invasive phenotype making them very successful in forming metastasis. This switch is controlled by the reciprocal expression of two master transcriptions factors: microphthalmia-associated transcription factor (MITF) controlling survival, differentiation and proliferation, and Brn-2 regulating invasiveness (Goodall et al., Cancer Res., 2008, 68: 7788-7794).

Numerous patient-derived melanoma cell lines exist that allow a large phenotype spectrum of proliferation and invasion to be tested. In order to determine whether TRPV2 expression level varies according to the invasive phenotype of the melanoma tumor, the present Inventors selected a subset of cell lines with gradual invasive potential and first quantified TRPV2 transcripts levels by quantitative RT-PCR compared to BRN2 being used as a well-defined marker for the invasive phenotype in melanoma (Thomson et al., Oncogene, 1995, 11: 691-700). Interestingly, while very low levels of TRPV2 mRNAs were found to be present in Normal Human Epithelial Melanocytes (NHEM), the quantity of detected TRPV2 transcripts was found to increase gradually in the non-invasive and 501mel cell line, WM793 (VGP), invasive WM266.4 derived from skin metastasis and reach a maximum in xenograft-derived highly invasive 451Lu cells (Figure 2A). This is paralleled by an increase in BRN2 mRNA expression and by an increment of the TRPV2 protein levels (Figure 2B). These observations revealed a positive correlation between TRPV2 expression levels and the invasive phenotype of the melanoma cell line. This result was confirmed by a functional approach where the amount of functional TRPV2 channels was probed using over-activation by an exogenous agonist. Ca²⁺ imaging experiments showed that application of 100 µM of cannabidiol (CBD), which has been used as a selective agonist of TRPV2 (Qin et al., J. Neurosci., 2008, 28: 6231-6238), induced a sustained intracellular (Ca²⁺) increase with amplitude and kinetics that corresponded to the amount of total TRPV2 channel present (Figure 2D) and further suggesting that TRPV2 is present at the surface of metastatic melanoma cells.

Plasma Membrane (PM) trafficking has been postulated to represent an important regulatory mechanism of TRPV2 activity. In order to evaluate TRPV2 membrane expression in melanoma cells, the present Inventors performed surface protein biotinylation experiments and observed that the plasma membrane population of TRPV2 channels increases gradually with the invasive potential of the cell line analyzed (Figure 2B). TRPV2 subcellular distribution was further evaluated by confocal immunofluorescence and showed similar results (Figure 2C). TRPV2 was mostly found to be expressed at the plasma membrane in the highly metastatic melanoma cell lines WM266.4 and 451Lu, as opposed to the non-invasive cell lines 501mel and WM793 displaying a preferential intracellular localization of TRPV2. It has been suggested that once addressed at the plasma membrane, TRPV2 is constituvely active, letting Ca²⁺ enter into the cell.

### TRPV2 controls resting calcium concentration in melanoma cells

To evaluate TRPV2 function in melanoma, the present Inventors over-expressed the TRPV2 channel in the non-invasive 501mel cell line or downregulated TRPV2 channel expression in two highly metastatic cell lines (WM266.4, 451Lu) using two short hairpin RNA (shRNA) sequences. Prior to functional studies, the successful overexpression or knockdown of TRPV2 was confirmed by Q-PCR (data not shown) and western blots experiments (Figure 3A). In addition, Ca²⁺ imaging experiments were performed to corroborate protein overexpression and suppression. The results obtained showed that GFP-TRPV2 transfected 501mel cells presented a greater response to CBD than the GFP expressing control cell lines (Figure 3B, left panel). In contrast, TRPV2 silencing in WM266.4 and 451Lu cells reduced Ca²⁺ influx in response to CBD (Figure 3B).

It has been shown that in most non-excitable cells, engagement of various plasma membrane receptors or mechanical stress stimulates TRPV2 activity by promoting its dynamic translocation from the endosomal compartment to the PM through a phosphatidylinositol-3 kinase (PI3K)-dependent pathway (Peralvarez-Marin et al., FEBS J., 2013, 280: 5471-5487). As some of the factors that are capable to recruit TRPV2 are present in serum, in normal growth conditions steady-state TRPV2-mediated Ca²⁺ influx might contribute to the resting intracellular Ca²⁺ concentration ([Ca²⁺]ᵢ). Basal TRPV2 channel activity was addressed using the Mn²⁺-quench technique. Interestingly, the rate of Mn2+-quench that was measured was smaller in the non-invasive 501mel cells compared to both metastatic cell lines. In addition, TRPV2 overexpression in 501mel cells was found to increase the Mn²⁺-flux while TRPV2 silencing in WM266.4 and 451Lu cells had the opposite effect (Figure 3 C-D). Thus, a population of TRPV2 channels is constituvely active in melanoma cells under normal growth condition and regulates resting Calcium levels.

### TRPV2 is critical for tumor cell migration and invasion but not for proliferation

Depending of the cellular context, TRPV2 has been shown to be specifically implicated in proliferation of tumor cells such as in glioblastoma cells (Nabissi et al., Carcinogenesis, 2010, 31: 794-803) or in the progression of tumor cells toward a more invasive phenotype, such as in in prostate and bladder cancers (Monet et al., Cancer Res, 2010, 70: 1225-1235; Caprodossi et al., Eur. Urol., 2008, 54: 612-620).

To determine how TRPV2 expression levels influence melanoma tumor progression, the present Inventors first evaluated whether TRPV2 activity affects melanoma proliferation and fount it to be dispensable at least for the highly invasive cells (data not shown). However, analysis of TRPV2 contribution to melanoma migration and invasion in the modified boyden chamber assay revealed that serum-induced migration and invasion were almost totally suppressed in the invasive melanoma cells when the protein was down-regulated. In contrast, TRPV2 overexpression in the non-invasive cells had the opposite effects, increasing the migratory and invasive capacity by 2.5 and 1.3 folds respectively, thus conferring the cells with a motile and invasive phenotype (Figure 4A). For the WM266.4 cell line, reductions in migration of 65.25% and of 79.69% were observed for shRNA1 and shRNA2, respectively; and reductions in invasion of 67.6% and of 64.64% were observed for shRNA1 and shRNA2, respectively. For the 451Lu cells, reductions in migration of 50.6% and of 39.4% were observed for shRNA1 and shRNA2, respectively; and reductions in invasion of 91.7% and of 94% were observed for shRNA1 and shRNA2 respectively.

To better understand the function of TRPV2 in melanoma cell migration, the present Inventors also performed individual cell tracking experiments over a 12-hour period of 2D-migration in the presence of a serum gradient. They chose to use the WM266.4 cell line because these cells spread out well on surface and display a morphology adapted for imaging studies. For direct comparison, these experiments were performed by seeding and recording the differentially labelled shRNA control and shRNA-V2-1 cell lines at the same time in order to place cells in the exact same conditions (data not shown). Under these conditions, they observed that although cell spreading and lamelipodial protrusions were overall not different between cell lines, control cells attached faster than shRNA TRPV2 cells (data not shown). Most importantly, they observed that cells transfected with a TRPV2 shRNA presented an altered cell directionality and motility relative to control cell lines (Figure 4B). Analysis of individual cell migration showed also that shRNA TRPV2 cells displayed a significantly reduced migration speed and traveled a shorter accumulated distance compared to control cell line (Figure 4C). To better mimic the *in situ* tumor-confined environment, tumor migration was further analyzed in 3-D. To do this, WM266.4 melanospheres were embedded in a collagen I matrix and cell invasion/migration followed by taking pictures every day. Corroborating the results obtained, downregulation of the TRPV2 was found to significantly reduce cell migration and invasion, as measured by the sphere outgrowth radius (Figure 4B).

Taken together, the results obtained clearly demonstrate that, by controlling intracellular Ca²⁺ homeostasis, TRPV2 potentiate the invasive behavior of metastatic melanoma tumor cells.

### TRPV2 associates with components of the acto-adhesive machinery

Cell migration is a sequential and interrelated multistep process involving the formation of lamellipodia/membrane protrusions at the front edge, cycles of adhesion and detachment, cell body contraction, and tail retraction (Ridley et al., 2003, Science, 2003, 302: 1704-1709). Focal adhesion turnover plays a pivotal role in cell migration. Small, nascent focal adhesions at the leading edge serve as traction points for the forces that move the cell body forward. On the other hand, disassembly of focal adhesions at the rear allows the retraction of the rear and net translocation of the cell in the direction of movement (Webb et al., Nat. Cell. Biol., 2002, 4: 97-100). To extravasate through capillary endothelium, cancer cells need to adhere to endothelial cells. During migration and invasion, the adhesions between cancer cells and the extracellular matrix also undergo disassembly (Webb et al., Nat. Cell. Biol., 2002, 4: 97-100).

In order to understand the mechanism by which TRPV2 promotes cell migration in melanoma, a potential association of TRPV2 and proteins from the focal adhesion (Fas) the cytoskeletal machinery was explored. To do this, the wild-type melanoma cells WM266.4 and 451Lu were seeded in fibronectin-coated cover-slips at low confluence, thus increasing the numbers of migrating cells. Under these conditions, TRPV2 was observed to be preferentially distributed at the leading edge of the cells and in some cases, a strong staining of TRPV2 was found near the lamella. Interestingly, in these spots, the TRPV2 signal was found to co-localize with cortactin (Figure 5A,c) and ZO-1 proteins (data not shown). TRPV2 was also found to partially co-localize with vimentin, vinculin and peripheral stress actin fibers (Figure 5A,a). Consistent with these results, reciprocal co-immunoprecipitation experiments performed on total cell lysates evidenced a physical interaction of TRPV2 with vinculin, cortactin and vimentin in the highly metastatic WM266.4 and 451Lu melanoma cell lines. It is also important to highlight that, the amount of TRPV2 or cortactin immunoprecipitates correlated well with the invasive potential of each cell line, suggesting a role of TRPV2 in invadosome formation or function.

### TRPV2 regulates cell migration through Calpain activation-dependent focal adhesion remodeling and actin stress fibers stabilization

To further investigate the mechanism by which TRPV2 controls cell migration in melanoma, the effect of manipulating TRPV2 expression on focal adhesion (FAs) dynamics was evaluate. FAs were detected following vinculin staining, a major component of these adhesion complexes, and their number was quantified. Overexpressing TRPV2 in the 501mel cells line was found to decrease by 23% the number of FAs, in contrast down-regulation of TRPV2 in the WM266.4 and 451Lu cell lines increased the number of FA compared to the control cell lines by 27% and 24%, respectively. Corroborating these results, transfection with a dominant negative (DN) form of TRPV2, increase the number of FAs by 60% with respect to the control 501mel cell line (Figure 6B).

Calpains are common intracellular Ca²⁺⁻ cysteine proteases implicated in the control of cell adhesion through focal adhesion disassembly by the cleavage of several structural FAs components. Thus, the present Inventors investigated basal calpain activation in the melanoma cell lines where TRPV2 was overexpressed or reduced. They observed that TRPV2 overexpression in the non-invasive 501mel melanoma cell line induced a 2-fold increase in basal calpain activity compared to the control cell line (Figure 6A). Moreover this activation was not significantly different if a dominant negative form of TRPV2 was overexpressed, thus suggesting that the activity of TRPV2 channels promotes calpain activation. Supporting these results, downregulation of TRPV2 in both invasive melanoma cells lines was found to reduce basal calpain activity by 50% (Figure 6A). These results suggest that TRPV2 activation at the vicinity of the FAs creates subplasmalemal calcium microdomains allowing localized and targeted activation of calpain and FA dissolution.

Besides FAs turnover, cell migration requires dynamic changes in the actin cytoskeleton. It has been suggested that TRPV2-activity might promotes actin assembly/disassembly (Link et al., Nat. Immunol. 2010, 11: 232-239). Therefore, to decipher further the molecular mechanism by which TRPV2 controls cell migration, the present Inventors sought to compare the actin-fibers dynamics in the melanoma cells bearing an overexpression or suppression of TRPV2 channels. For this, cells seeded on fibronectin-coated coverslips were stained with SiR-actin, a fluorogenic probe permeable to the cell membrane and highly specific for F-actin proteins, and the number of stress fibers and amount of actin assembly was evaluated and normalized to the size of the cells. They found that, although there was no difference in the number cells with actin fiber polymerization, the amount of actin bundles was increased in cells overexpressing TRPV2 (data not shown). Analogously, cells presenting a down-regulation in the TRPV2 levels presented a reduction in actin polymerization (data not shown). In support of these results, they found that suppression of TRPV2 activity, by transfecting cells with a DN constructs or by suppressing TRPV2 expression, induced cofilin dephosphorylation on serine 3. Cofilin is an essential regulator of actin filament dynamics, being inactivated by phosphorylation and reactivated by dephosphorylation. Once activated, cofilin can bind to actin filaments and stimulate their depolymerization leading to their dissolution. Thus, this might suggest that TRPV2 basal signaling might contribute to actin stabilization by promoting cofilin phosphorylation. In addition, they observed by time-lapse video microscopy that TRPV2 stimulation, in response to CBD, promoted a strong increase in actin polymerization (data not shown). It has been recently shown that intracellular Ca²⁺ increments promote actin assembly leading to an improved melanoma cell migration (Baljinnyam et al., Cancer Res., 2010, 70: 5607-5617).

### Downregulation of TRPV2 expression prevents metastatic formation in vivo

The study was then extended to *in vivo* models. Using an experimental model of metastasis formation in xenotransplanted zebrafish, the role of TRPV2 in melanoma metastasis formation was evaluated *in vivo.* Zebrafish larvae do not develop a proper adaptive immune system until late maturation stages, thus human cells can survive and metastasize when transplanted into zebrafish larvae for the first two weeks after being born. This makes this model a fast and inexpensive tool to evaluate the metastatic potential of cell lines. For these experiments, the 451Lu cell line was selected as it is used extensively *in vivo* for its high metastatic potential. Both the shCtrl cell line and the shRNA TRPV2 cell line were labelled with the GFP protein. In order to be able to evaluate the difference between the two cell lines, the control cell line was labelled with the red fluorescence dye, CmDil, and both cell lines were co-injected in the duct of cuvier of 48 hours old zebrafish embryos. Two (2) days post transplantation, only cells from the control cell line, recognizable by their double labelling, were disseminated through the fish body and only rarely a singly labelled cell corresponding to the shRNA TRPV2 transfected cell line (data not shown) could be observed.

### TRPV2 is overexpressed in patient with malignant and metastatic melanoma

To analyze the expression of TRPV2 in patients with melanoma, a panel of 100 samples from a tumor microarray was stained using a specific and well characterized antibody for TRPV2 and the corresponding isotypic control. The samples originated from 62 patients with malignant melanoma (Grade I: 27; Grade II: 29; Grade III: 4; Grade IV: 2), 20 patients with metastatic malignant melanoma and 18 nevi samples from healthy subjects. From this panel, a strong difference in TRPV2 expression was observed between normal subjects and patients with melanoma (Figure 7A,B, left panel). A significant difference in TRPV2 staining was also observed in patients with metastatic melanoma compared to patients with malignant melanoma, as well as samples derived from the skin and those coming from the lymph nodes (Figure 7B, middle and right panel). However, due to the limited number of samples, it was not possible to find a difference in TRPV2 expression between the different melanoma stages, but an increased expression in people older than 35 years was observed. Taken together, these results validate the hypothesis that TRPV2 overexpression in melanoma promotes cell migration and metastasis.

### Conclusion

In the present study, the present Inventors report four major findings for understanding the relation between melanoma metastasis and Ca²⁺ signalling. For the first time, they have shown that the TRPV2 channel is strongly expressed in multiple melanoma cell lines, representing the major Ca²⁺ channel involved in cell migration. More importantly, the results obtained were corroborated in human melanoma tissues where an important difference in the expression of TRPV2 protein levels was observed between melanoma samples and normal tissue samples. Second, they have shown that TRPV2 expression levels positively correlated with the invasive phenotype of the cell lines/tumors. Third, they have presented evidence that TRPV2 overexpression in a non-invasive melanoma cell line confers the cell line with an invasive phenotype both *in vitro* and *in vivo.* In contrast, the invasive and migratory potential of metastatic melanoma cells was reduced upon TRPV2 silencing. Finally, they presented evidence suggesting that TRPV2-dependent calcium signalling controls focal adhesion turnover and actin cytoskeleton dynamics controlling melanoma migration and invasion.

## Claims

1. An *in vitro* method for diagnosing melanoma in a subject comprising steps of:
(a) determining the expression level of TRPV2 in a biological sample obtained from the subject; and
(b) comparing the expression level of TRPV2 measured in (a) with the expression level of TRPV2 measured in a biological sample obtained from at least one healthy subject,
wherein an increased expression level of TRPV2 in the subject compared to the healthy subject is indicative of melanoma

2. The *in vitro* method according to claim 1, further comprising a step of:
(c) comparing the expression level of TRPV2 measured in (a) with the expression level of TRPV2 measured in a biological sample obtained from at least one control subject diagnosed with non-metastatic melanoma,
wherein an increased expression level of TRPV2 in the subject compared to the control subject is indicative of aggressive/invasive or metastatic melanoma.

3. An *in vitro* method for diagnosing aggressive/invasive or metastatic melanoma in a subject comprising steps of:
(a) determining the expression level of TRPV2 in a biological sample obtained from the subject; and
(b) comparing the expression level of TRPV2 measured in (a) with the expression level of TRPV2 measured in a biological sample obtained from at least one control subject diagnosed with non-metastatic melanoma,
wherein an increased expression level of TRPV2 in the subject compared to the control subject is indicative of aggressive/invasive or metastatic melanoma.

4. An *in vitro* method for distinguishing between non-metastatic melanoma and metastatic melanoma in a subject or for determining the aggressiveness of melanoma in a subject, said method comprising steps of:
(a) determining the expression level of TRPV2 in a biological sample obtained from the subject;
(b) comparing the expression level of TRPV2 measured in (a) with the expression level of TRPV2 measured in a biological sample obtained from at least one healthy subject; and
(c) comparing the expression level of TRPV2 measured in (a) with the expression level of TRPV2 measured in a biological sample obtained from at least one control subject diagnosed with non-metastatic melanoma,
wherein an increased expression level of TRPV2 in the subject compared to the control subject is indicative of aggressive/invasive or metastatic melanoma, and wherein an expression level of TRPV2 in the subject comprised between the expression level of TRPV2 in the healthy subject and the expression level of TRPV2 in the control subject is indicative of non-metastatic melanoma.

5. The *in vitro* method according to any one of claims 1 to 4, wherein the biological sample is a skin biopsy sample or a blood sample containing circulating melanoma cells.

6. The *in vitro* method according to any one of claims 1 to 5, wherein determining the expression level of TRPV2 in a biological sample includes measuring the amount of TRPV2 mRNA in the sample or determining the amount of TRPV2 protein in the sample.

7. A reagent that specifically reacts with TRPV2 mRNA or protein for use in the *in vitro* diagnosis of melanoma or for use in the *in vitro* diagnosis of aggressive/invasive or metastatic melanoma.

8. A reagent that specifically reacts with TRPV2 mRNA or protein for the use according to claim 7, wherein said reagent is selected from the group consisting of oligonucleotide probes that specifically hybridize to TRPV2 mRNA transcripts, oligonucleotide primers that specifically amplify TRPV2 mRNA transcripts, antibodies that specifically recognize/bind the TRPV2 protein, and TRPV2-binding peptides that specifically bind to the TRPV2 protein.

9. A reagent that specifically reacts with TRPV2 mRNA or protein for the use according to claim 8, wherein said reagent is labeled with a detectable moiety.

10. A kit for use in the *in vitro* diagnosis of melanoma or for the *in vitro* diagnosis of aggressive/invasive or metastatic melanoma, said kit comprising a reagent that specifically reacts with TRPV2 mRNA or protein and instructions to perform a diagnosis according to a method according to any one of claims 1 to 6.

11. A kit for the use according to claim 10, wherein the reagent that specifically reacts with TRPV2 mRNA or protein is selected from the group consisting of oligonucleotide probes that specifically hybridize to TRPV2 mRNA transcripts, oligonucleotide primers that specifically amplify TRPV2 mRNA transcripts, antibodies that specifically recognize/bind the TRPV2 protein, and TRPV2-binding peptides that specifically bind to the TRPV2 protein.

12. A TRPV2 therapeutic agent, or a pharmaceutical composition thereof, for use in the treatment of melanoma, wherein the TRPV2 therapeutic agent is selected among TRPV2 agonists or activators and TRPV2-binding peptides and antibodies conjugated to a therapeutic moiety.

13. A TRPV2 therapeutic agent, or a pharmaceutical composition thereof, for the use according to claim 12, wherein the therapeutic moiety is selected from the group consisting of anti-cancer drugs, nano-metallic clusters, boron clusters, anti-inflammatory agents, immunomodulatory agents, analgesics, antimicrobial agents, antibacterial agents, antibiotics, antioxidants, antiseptic agents, and combinations thereof.

14. A TRPV2 therapeutic agent, or a pharmaceutical composition thereof, for use in the prevention of melanoma metastasis formation, wherein the TRPV2 therapeutic agent is selected among TRPV2 antagonists and RNA interference agents.

15. A pharmaceutical composition for the use according to any one of claims 12 to 14, wherein said pharmaceutical composition comprises a therapeutically effective amount of the TRPV2 therapeutic agent and at least one pharmaceutically acceptable carrier or excipient.
